# EUROPEAN PATENT APPLICATION

(11) **EP 4 275 758 A2**
(43) Date of publication of application: **15.11.2023**
(21) Application number: 23184312.9
(22) Date of filing: 26.10.2021
(51) Int. Cl.: A61P 35/00

(54) **METHODS OF FUNCTIONALIZING NANOPARTICLES**

(30) Priority: 27.10.2020 US 202063105995 P; 20.11.2020 US 202063116393 P; 23.11.2020 US 202063117110 P; 01.03.2021 US 202163155043 P; 15.07.2021 US 202163222181 P; 09.09.2021 US 202163242201 P; 12.10.2021 US 202163254837 P
(62) Divisional of application: 21811587.1
(71) Applicant: Elucida Oncology, Inc., Monmouth Junction, NJ 08852 (US)
(72) Inventor: MA, Kai, Montgomery, NJ 08852 (US); TÜRKER, Melik Ziya, Princeton, NJ 08542 (US); WU, Fei, Plainsboro, NJ 08852 (US); CHEN, Feng, Princeton, NJ 08540 (US); GARDINIER II, Thomas Courtney, Raritan, NJ 08869 (US); VENKATESAN, Aranapakam M., Rego Park, NY 11374 (US); GERMANO Jr., Geno J., Philadelphia, PA 19118 (US)
(74) Representative: Garner, Stephen

(57) **Abstract**

This disclosure relates to methods of functionalizing a nanoparticle, e.g., for conjugation to a targeting ligand and/or payload moiety, such as for the production of a nanoparticle drug conjugate (NDC).

## Description

### RELATED APPLICATIONS

This application claims the benefit of U.S. Provisional Application No. 63/105,995, filed on October 27, 2020, U.S. Provisional Application No. 63/116,393, filed on November 20, 2020, U.S. Provisional Application No. 63/117,110, filed on November 23, 2020, U.S. Provisional Application No. 63/155,043, filed on March 1, 2021, U.S. Provisional Application No. 63/222,181, filed on July 15, 2021, U.S. Provisional Application No. 63/242,201, filed on September 9, 2021, and U.S. Provisional Application No. 63/254,837, filed on October 12, 2021, the contents of which are each incorporated herein by reference in their entireties.

### BACKGROUND

Nanoparticle drug conjugates (NDCs) offer the potential to resolve many of the shortcomings of other drug carrier platforms, such as antibody drug conjugates (ADCs). For example, NDCs with an ultrasmall size (e.g., a diameter of 20 nm or less) can pass through disrupted blood brain barriers and deliver therapeutics to brain tumors, that is not possible using other platforms such as ADCs. Ultrasmall nanoparticles can exhibit deep tumor penetration and provide homogeneous delivery of therapeutic molecules to tumors, while other drug carrier platforms such as ADCs have limited tumor penetration due to slow intratumoral diffusion. NDCs can also carry significantly more drug molecules than conventional drug delivery platforms such as ADCs, which allows NDCs to deliver a relatively larger amount of drugs to cancer cells. This is particularly useful when targeting cancer cells with low receptor expression, that complicate delivery of a sufficient amount of drug. Another advantage of NDCs is the possibility of coating the nanoparticle with an organic polymer layer, e.g., coating the nanoparticle surface with a layer of PEG groups, which can prevent adsorption of serum proteins to the nanoparticle in a physiological environment (e.g., in a subject), and may facilitate efficient urinary excretion and decrease aggregation of the nanoparticle (*see, e.g.,* Burns et al. "Fluorescent silica nanoparticles with efficient urinary excretion for nanomedicine", Nano Letters (2009) 9(1):442-448).

However, there are significant obstacles in the development and manufacture of NDCs. For example, it is very challenging to create NDCs that meet the stringent criteria in manufacturing controls, stability, drug release, safety, and efficacy required for clinical translation. In particular, creating a linkage between conjugated molecules (e.g., drug molecules and/or targeting ligands) and the nanoparticle carrier that meet these criteria is especially difficult.

As such, there is an unmet need for methods to functionalize nanoparticles and permit conjugation of molecules to the nanoparticle, such as drug molecules or targeting ligands.

### SUMMARY

The present disclosure features methods of functionalizing a nanoparticle. The method can include preparation of nanoparticles suitable for conjugating compounds to the nanoparticle surface (e.g., targeting ligands, such as cancer targeting ligands, e.g., folate receptor targeting ligands, e.g., folic acid; and therapeutic agents, such as cytotoxic compounds, e.g., exatecan) to form nanoparticle drug conjugates (NDCs). The resulting NDCs exhibit both highly stable linkage of the conjugated molecule to the nanoparticle, and provide effective drug release in targeted biological systems, *e.g.,* in cancer cells.

The synthetic method disclosed herein can involve a sequence of reactions, which can introduce a series of different reactive groups (sometimes referred to herein as functional groups) or compounds (e.g., payload moieties, or targeting ligands) to the nanoparticle surface. These reactions can include silane condensation, Diels-Alder reaction, and "Click Chemistry," 2+3 or 2+4 cycloaddition reactions, among others, e.g., other reactions described herein. The method may be used to functionalize ultrasmall nanoparticles, including nanoparticles that are coated with an organic polymer, and/or silica nanoparticles (e.g., ultrasmall PEGylated silica nanoparticles, such as C'Dots).

The method of functionalizing a nanoparticle can comprise a step of contacting a nanoparticle with a first bifunctional precursor, such as a bifunctional precursor comprising a silane moiety and another reactive group (e.g., a diene, an amine, a thiol, a hydroxyl, an azide, an alkene, or an alkyne) wherein the nanoparticle comprises a surface that is reactive with the silane (e.g., a silica surface), and wherein the contacting is under conditions suitable for reaction between the silane moiety and the nanoparticle surface (e.g., conditions described herein suitable for silane condensation), thereby forming a covalent bond between the silane moiety and a surface of the nanoparticle, and providing a nanoparticle functionalized with the reactive group (e.g., a diene, an amine, a thiol, a hydroxyl, an azide, an alkene, or an alkyne). It will be understood that this method can be used to provide a nanoparticle comprising a plurality of the reactive groups.

The contacting of the nanoparticle with the first bifunctional precursor may involve the insertion of the bifunctional precursor into the interstitial space between organic polymer molecules (e.g., PEG chains) attached to the nanoparticle, such as when using a nanoparticle comprising a surface coated with PEG. As such, this method overcomes difficulties in conventional methods of nanoparticle functionalization that cannot effectively deliver precursor molecules to the surface of a nanoparticle comprising an organic polymer layer (e.g., a layer of PEG molecules) for functionalization.

The method disclosed herein may further comprise contacting the nanoparticle functionalized with the reactive group (e.g., the nanoparticle functionalized with a diene, an amine, a thiol, a hydroxyl, an azide, an alkene, or an alkyne) with a second bifunctional precursor, wherein the second bifunctional precursor comprises a group that is reactive with the reactive group on the nanoparticle, and wherein the second bifunctional precursor comprises another functional group (e.g., an alkyne moiety (e.g., dibenzoazacyclooctyne (DBCO)), a diene, an amine, a thiol, a hydroxyl, an alkene, or an azide). For example, the second bifunctional precursor may comprise a dienophile (e.g., maleimide) that is reactive with the reactive group on the nanoparticle (e.g., a diene), and the second bifunctional precursor may also comprise an alkyne moiety (e.g., DBCO). The contacting may be under conditions suitable for a reaction between the functionalized nanoparticle and the second bifunctional precursor (e.g., reaction conditions disclosed herein), thereby covalently bonding the second bifunctional precursor to the nanoparticle and providing a nanoparticle functionalized with the another functional group (e.g., an alkyne moiety, such as DBCO). It will be understood that the method can be used to provide a nanoparticle functionalized with a plurality of the another functional group (e.g., a plurality of DBCO moieties).

The method may further comprise contacting the nanoparticle functionalized with the another functional group (e.g., a DBCO functionalized nanoparticle) with a compound comprising a group that is reactive with the another functional group on the nanoparticle. For example, the compound may comprise an alkyne-reactive group (e.g., an azide, diene, nitrone, or nitrile oxide), suitable for reacting with an alkyne group of an alkyne-functionalized nanoparticle. The contacting may be under conditions suitable for a reaction between the alkyne moiety and the alkyne-reactive group, e.g., reaction conditions described herein, such as Click Chemistry conditions, or other cycloaddition conditions (e.g., 3+2 cycloaddition or 4+2 cycloaddition), thereby forming a nanoparticle functionalized with the compound. The compound comprising a group that is reactive with the functional group on the nanoparticle may comprise a payload moiety (e.g., a cytotoxic drug disclosed herein, such as exatecan) or a targeting ligand (e.g., a folate receptor targeting ligand, such as folic acid). The method may be used to covalently attach a plurality of compounds to the nanoparticle surface. For example, the method may introduce a plurality of the compound comprising a group that is reactive with the functionalized nanoparticle, or introduce a plurality of different compounds each comprising a group that is reactive with the functionalized nanoparticle (e.g., a plurality of targeting ligands, a plurality of payload moieties, or a combination thereof), and covalently attaching said compounds to the nanoparticle.

It will be understood that each step of the method may be used to introduce a plurality of functional groups to the nanoparticle. For example, the nanoparticle can be contacted with a plurality of the first bifunctional precursor comprising a silane group and a reactive group, providing a nanoparticle functionalized with a plurality of reactive groups (e.g., a plurality of diene moieties). The nanoparticle functionalized with a plurality of reactive groups (e.g., a plurality of diene moieties) can be contacted with a plurality of second bifunctional precursors, wherein the second bifunctional precursor comprises a group that is reactive with the reactive group on the nanoparticle, and wherein the second bifunctional precursor comprises another functional group (e.g., an alkyne, e.g., DBCO), thereby providing a nanoparticle functionalized with a plurality of the functional groups from the second bifunctional precursor (e.g., a plurality of DBCO moieties). The nanoparticle functionalized with a plurality of the functional groups from the second bifunctional precursor may then be contacted with a plurality of compounds comprising a group that is reactive with the functional group on the nanoparticle, providing a nanoparticle comprising a plurality of the compounds. The nanoparticle functionalized with a plurality of the functional groups from the second bifunctional precursor may be contacted with a first plurality of compounds comprising a group that is reactive with the functional group on the nanoparticle (e.g., a targeting ligand comprising an azide), and may subsequently be contacted with a second plurality of compounds comprising a group that is reactive with the functional group on the nanoparticle (e.g., a payload-linker conjugate comprising an azide), wherein the first and second pluralities comprise structurally distinct compounds, thereby providing a nanoparticle comprising two distinct pluralities of compounds (e.g., a plurality of targeting ligands, and a plurality of payload moieties).

For example, the method can comprise forming a nanoparticle functionalized with a plurality of diene moieties (e.g., cyclopentadiene moieties), e.g., by contacting a nanoparticle with a plurality of first bifunctional precursors comprising a silane moiety and a diene moiety, under conditions sufficient for a reaction between a silane moiety and a surface of the nanoparticle. The method can comprise forming a nanoparticle functionalized with a plurality of alkyne moieties (e.g., DBCO moieties), e.g., by contacting a nanoparticle comprising a plurality of diene moieties (e.g., cyclopentadiene moieties) with a plurality of second bifunctional precursors comprising a dienophile (e.g., maleimide) and an alkyne moiety (e.g., DBCO), under conditions sufficient for a reaction between a diene moiety and a dienophile. The method may further comprise: (a) reacting a first portion of the plurality of alkyne moieties on a nanoparticle with a first compound comprising an alkyne-reactive group (under conditions sufficient for a reaction between an alkyne moiety and an alkyne-reactive group); and (b) reacting a second portion of the plurality of alkyne moieties on a nanoparticle with a second compound comprising an alkyne-reactive group (under conditions sufficient for a reaction between an alkyne moiety and an alkyne-reactive group), thereby forming nanoparticle functionalized with a plurality of the first compound and a plurality of the second compound, wherein the first compound and the second compound are chemically distinct. For example, (a) the first compound may be a compound of Formula (D) (e.g., a compound of Formula (D-1)) disclosed herein; and the second compound may be a compound of Formula (E) (e.g., a compound of Formula (E-1)), disclosed herein. Alternatively, the first compound may be a compound of Formula (E) (e.g., a compound of Formula (E-1)) disclosed herein, and the second compound is a compound of Formula (D) (e.g., a compound of Formula (D-1)) disclosed herein.

The present disclosure is also directed to a method of functionalizing a silica nanoparticle, comprising: (i) contacting a silica nanoparticle with a first bifunctional precursor, wherein the first bifunctional precursor comprises a silane moiety and a cyclopentadiene moiety, wherein the silica nanoparticle comprises a surface that is reactive with the silane moiety (e.g., a silica surface), and wherein the contacting is under conditions suitable for reaction between the silane moiety and the silica nanoparticle surface, thereby forming a covalent bond between the silane moiety and a surface of the nanoparticle, and providing a nanoparticle functionalized with a cyclopentadiene moiety; (ii) contacting the nanoparticle functionalized with a cyclopentadiene moiety with a second bifunctional precursor, wherein the second bifunctional precursor comprises an alkyne moiety (e.g., DBCO) and a dienophile (e.g., maleimide), wherein the contacting is under conditions suitable for a reaction between the dienophile and the cyclopentadiene moiety, thereby reacting the diene moiety of the nanoparticle with the second bifunctional precursor, and providing a nanoparticle functionalized with an alkyne moiety; and (iii) contacting the alkyne moiety with a compound comprising an azide moiety, under conditions suitable for a reaction between the alkyne moiety and azide moiety (e.g., click chemistry conditions), thereby reacting the alkyne moiety of the nanoparticle with the azide moiety and providing a nanoparticle functionalized with the compound (e.g., a compound comprising a payload or a targeting ligand).

The present disclosure is also directed to a A method of functionalizing a silica nanoparticle, comprising: (i) contacting a silica nanoparticle with a first bifunctional precursor comprising a structure of Formula (A), thereby providing a nanoparticle functionalized with a cyclopentadiene moiety; (ii) contacting the nanoparticle functionalized with a cyclopentadiene moiety with a second bifunctional precursor comprising a structure of Formula (B), thereby providing a nanoparticle functionalized with an alkyne moiety; and (iii) contacting the alkyne moiety with a compound of Formula (D) or Formula (E), or both, thereby providing a nanoparticle functionalized with a targeting ligand, a payload moiety, or both.

The first bifunctional precursor may comprise the structure of Formula (A-1) provided herein. The second bifunctional precursor may comprise a structure of Formula (B-1) provided herein. The compound of Formula (D) may comprise a structure of Formula (D-1) provided herein. The compound of Formula (E) may comprise a structure of Formula (E-1) provided herein.

The methods disclosed herein can provide a nanoparticle comprising a compound of Formula (NP-2) provided herein. The average nanoparticle to (NP-2) ratio may be from about 1:1 to about 1:50 (each nanoparticle comprises an average of 1-50 units of NP-2), e.g., about 1:40, about 1:30, about 1:25, about 1:20, about 1:15, about 1:14, about 1:13, about 1:12, about 1:11, or about 1:10.

The method can provide a nanoparticle comprising a compound of Formula (NP-3) provided herein. The average nanoparticle to (NP-3) ratio may be from about 1:1 to about 1:80 (each nanoparticle comprises an average of 1-80 units of NP-3), e.g., about 1:60, about 1:40, about 1:30, about 1:28, about 1:26, about 1:25, about 1:24, about 1:23, about 1:22, about 1:21, about 1:20, about 1:19, or about 1:18.

The method can provide a nanoparticle comprising a compound of Formula (NP-2) and a compound of Formula (NP-3), the structures of which are provided herein, wherein the ratio of nanoparticle:(NP-3):(NP-2) is about 1:20:10, 1:20:11, 1:20:12, 1:20:13, 1:20:14, 1:20:15, 1:21:10, 1:21:11, 1:21:12, 1:21:13, 1:21:14, 1:21:15, 1:22:10, 1:22:11, 1:22:12, 1:22:13, 1:22:14, 1:22:15, 1:23:10, 1:23:11, 1:23:12, 1:23:13, 1:23:14, 1:23:15, 1:24:10, 1:24:11, 1:24:12, 1:24:13, 1:24:14, 1:24:15, 1:25:10, 1:25:11, 1:25:12, 1:25:13, 1:25:14, or 1:25:15.

An advantage of the methods disclosed herein is the use of relatively stable precursors (e.g., diene-silane precursors), resulting in stable functionalized nanoparticles. For example, functionalized nanoparticles (e.g., NDCs) that may be produced using the methods disclosed herein can avoid premature or undesired cleavage, which can occur in functionalized nanoparticles (e.g., NDCs) produced using other methods or with other precursors. For example, other methods of functionalizing nanoparticles employ precursors that result in nanoparticles with reactive moieties on the surface of the nanoparticle, that can promote undesired reactivity that may lead to, *e.g.,* premature release of a payload or undesired release of the targeting ligand. Additionally, other methods of nanoparticle functionalization use precursors that are unstable and can self-condense during reaction, causing undesired aggregation. The aggregates can be very difficult to separate from the functionalized nanoparticles.

In contrast, the methods disclosed herein can employ relatively stable precursors, and the resulting functionalized nanoparticle (e.g., NDCs) are stable and highly pure. For example, the functionalized nanoparticles of the present disclosure can be prepared with a diene-silane precursor (such as a cyclopentadiene-silane precursor), to afford a nanoparticle functionalized with one or more diene groups. The diene groups may then be reacted with a second precursor, such as a dienophile-containing precursor (e.g., a PEG-maleimide derivative, e.g., a DBCO-PEG-maleimide), causing a stable cycloadduct to form. The resulting functionalized nanoparticle, comprising the cycloadduct, may optionally be reacted with one or more subsequent precursors (such as a targeting ligand precursor and/or a payload-linker conjugate precursor described herein), to further functionalize the nanoparticle. The diene-silane precursor, and the cycloadducts that are produced, do not exhibit undesired characteristics that can be found in other functionalized nanoparticles or their precursors. For example, the functionalized nanoparticles (e.g., NDCs) prepared using the methods disclosed herein have relatively high serum stability, and can be produced in high yield and purity (e.g., free of aggregated precursor). Additionally, as this approach is highly modular, any desired ratio of payload, targeting ligand, or otherwise, can be introduced to the nanoparticle. Examples of preparing nanoparticles using these methods, and their benefits, are provided in the Examples.

An exemplary sequence of functionalizing a nanoparticle is depicted in Scheme 1.

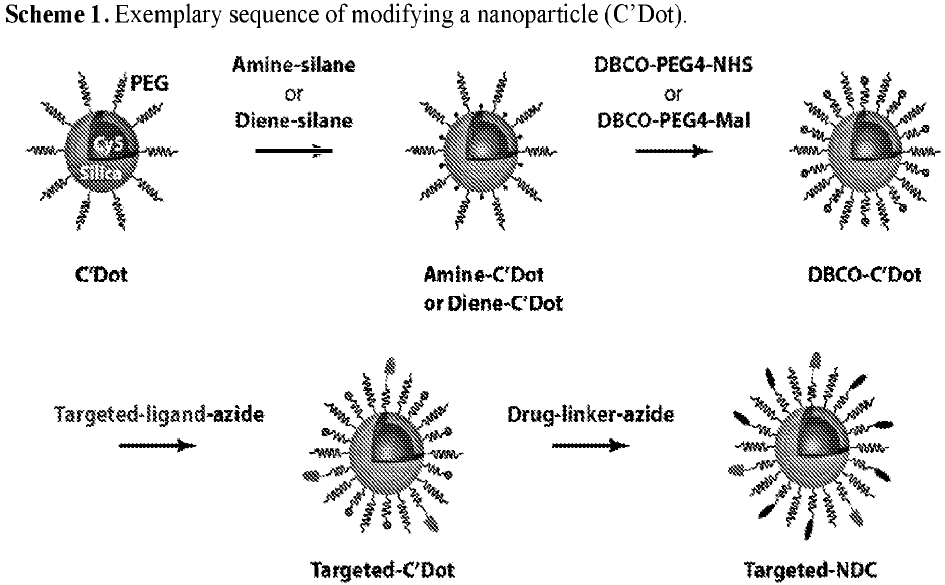

### BRIEF DESCRIPTION OF DRAWINGS

**FIG. 1****.** illustrates the structure of an exemplary nanoparticle drug conjugate (NDC) that can be prepared using the methods disclosed herein.
**FIG. 2** is a graph comparing efficiency between different approaches for functionalizing nanoparticles with DBCO moieties.

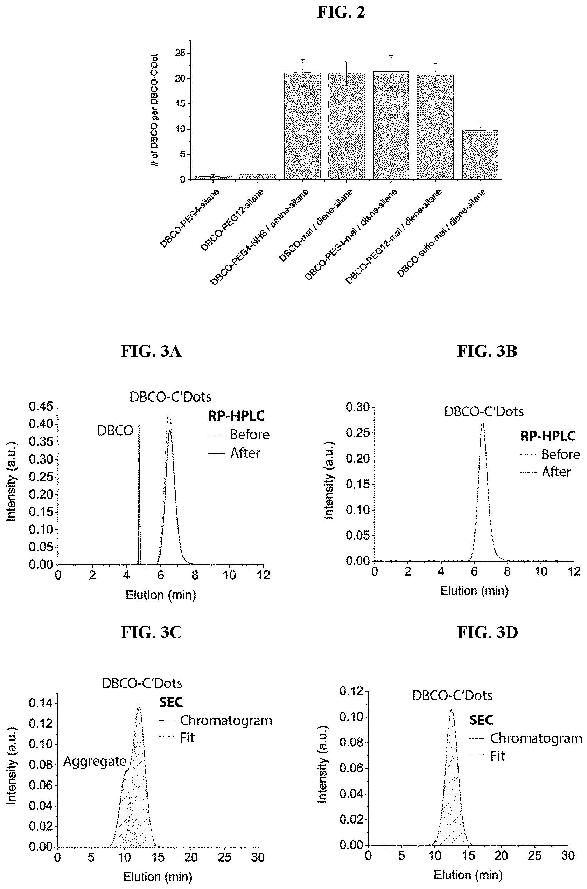

**FIGS. 3A-3D** are RP-HPLC and SEC chromatograms. **FIG. 3A** provides the RP-HPLC chromatogram of DBCO-functionalized nanoparticles prepared using an amine-based bifunctional precursor, before and after incubation in PBS for 24 hours. **FIG. 3B** provides the RP-HPLC chromatogram of DBCO-functionalized nanoparticles prepared using a diene-based bifunctional precursor, before and after incubation in PBS for 24 hours. **FIG. 3C** provides the SEC chromatogram of DBCO-functionalized nanoparticles prepared using an amine-based bifunctional precursor. **FIG. 3D** provides the SEC chromatogram of DBCO-functionalized nanoparticles prepared using a diene-based bifunctional precursor.
**FIG. 4** is a spectrograph from fluorescence correlation spectroscopy (FCS) of an exemplary NDC prepared using the methods disclosed herein, indicating the average hydrodynamic diameter.
**FIG. 5** shows overlaid UV-Vis spectra of an exemplary functionalized nanoparticle at different stages of functionalization, indicating characteristic absorption peaks that can be used to verify the presence and average number of each conjugate (e.g., targeting ligand, e.g., folic acid (FA), or payload, e.g., exatecan) per particle.
**FIGS. 6A- 6B** provide the RP-HPLC chromatogram (**FIG. 6A**) and SEC chromatogram (**FIG. 6B**) at two wavelengths of an exemplary NDC prepared using methods disclosed herein.
**FIG. 7** is a graph providing the amount of drug (exatecan) released over time from an exemplary NDC while incubating with cathepsin-B at 37 °C. The inset provides the overlaid UV-Vis spectra recorded at each time point.
**FIGS. 8A-8B** are graphs illustrating the serum stability of exemplary NDCs prepared using methods disclosed herein. **FIG. 8A** compares the stability of an NDC produced using a diene-based bifunctional precursor and an NDC produced using an amine-based bifunctional precursor in 10% human serum at 37 °C, over 7 days. **FIG. 8B** compares the stability of an NDC produced using a diene-based bifunctional precursor and an NDC produced using an amine-based bifunctional precursor in 10% mouse serum at 37 °C, over 7 days.

### DETAILED DESCRIPTION

### Selected Chemical Definitions

As used herein, the term "alkyl" refers to monovalent aliphatic hydrocarbon group that may comprise 1 to 18 carbon atoms, such as 1 to about 12 carbon atoms, or 1 to about 6 carbon atoms ("C₁₋₁₈ alkyl"). An alkyl group can be straight chain, branched chain, monocyclic moiety or polycyclic moiety or combinations thereof. Examples of alkyl groups include methyl, ethyl, propyl, isopropyl, butyl, *iso*-butyl, *sec*-butyl, *tert*-butyl, pentyl, hexyl, cyclopropyl, cyclobutyl, cyclopentyl, cyclohexyl, norbornyl, and the like. Each instance of an alkyl group may be independently optionally substituted, i.e., unsubstituted (an "unsubstituted alkyl") or substituted (a "substituted alkyl") with one or more substituents e.g., for instance from 1 to 5 substituents, 1 to 3 substituents, or 1 substituent.

As used herein, the term "alkenyl" refers to a monovalent straight-chain or branched hydrocarbon group having from 2 to 18 carbon atoms, one or more carbon-carbon double bonds, and no triple bonds ("C₂₋₁₈ alkenyl"). An alkenyl group may have 2 to 8 carbon atoms, 2 to 6 carbon atoms, 2 to 5 carbon atoms, 2 to 4 carbon atoms, or 2 to 3 carbon atoms. The one or more carbon-carbon double bonds can be internal (such as in 2-butenyl) or terminal (such as in 1-butenyl). Examples of alkenyl groups include ethenyl, 1-propenyl, 2-propenyl, 1-butenyl, 2-butenyl, butadienyl, pentenyl, pentadienyl, hexenyl, heptenyl, octenyl, octatrienyl, and the like. Each instance of an alkenyl group may be independently optionally substituted, i.e., unsubstituted (an "unsubstituted alkenyl") or substituted (a "substituted alkenyl") with one or more substituents e.g., for instance from 1 to 5 substituents, 1 to 3 substituents, or 1 substituent.

As used herein, the term "alkynyl" refers to a monovalent straight-chain or branched hydrocarbon group having from 2 to 18 carbon atoms, and one or more carbon-carbon triple bonds ("C₂₋₁₈ alkynyl"). The alkynyl group may have 2 to 8 carbon atoms, 2 to 6 carbon atoms, 2 to 5 carbon atoms, 2 to 4 carbon atoms, or 2 to 3 carbon atoms. The one or more carbon-carbon triple bonds can be internal (such as in 2-butynyl) or terminal (such as in 1-butynyl). Examples of alkynyl groups include ethynyl, 1-propynyl, 2-propynyl, 1-butynyl, 2-butynyl, and the like. Each instance of an alkynyl group may be independently optionally substituted, i.e., unsubstituted (an "unsubstituted alkynyl") or substituted (a "substituted alkynyl") with one or more substituents, e.g., for instance from 1 to 5 substituents, 1 to 3 substituents, or 1 substituent.

As used herein, the term "heteroalkyl" refers to a non-cyclic stable straight or branched chain, or combinations thereof, including at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any position of the heteroalkyl group.

The terms "alkylene," "alkenylene," "alkynylene," or "heteroalkylene," alone or as part of another substituent, mean, unless otherwise stated, a divalent radical derived from an alkyl, alkenyl, alkynyl, or heteroalkyl, respectively. The term "alkenylene," by itself or as part of another substituent, means, unless otherwise stated, a divalent radical derived from an alkene. An alkylene, alkenylene, alkynylene, or heteroalkylene group may be described as, e.g., a C₁₋₆-membered alkylene, C₁₋₆-membered alkenylene, C₁₋₆-membered alkynylene, or C₁₋₆-membered heteroalkylene, wherein the term "membered" refers to the non-hydrogen atoms within the moiety. In the case of heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- may represent both -C(O)₂R'- and -R'C(O)₂-. Each instance of an alkylene, alkenylene, alkynylene, or heteroalkylene group may be independently optionally substituted, *i.e.,* unsubstituted (an "unsubstituted alkylene") or substituted (a "substituted heteroalkylene") with one or more substituents.

As used herein, the terms "substituted alkyl," "substituted alkenyl," "substituted alkynyl," "substituted heteroalkyl," "substituted heteroalkenyl," "substituted heteroalkynyl," "substituted cycloalkyl," "substituted heterocyclyl," "substituted aryl," and "substituted heteroaryl" refer to alkyl, alkenyl, alkynyl, heteroalkyl, heteroalkenyl, heteroalkynyl, cycloalkyl, heterocyclyl, aryl, and heteroaryl moieties, respectively, having substituents replacing one or more hydrogen atoms on one or more carbons or heteroatoms of the moiety. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, e.g., with the substituents described above.

As used herein, the term "alkoxy" refers to a group of formula -O-alkyl. The term "alkoxy" or "alkoxyl" includes substituted and unsubstituted alkyl, alkenyl and alkynyl groups covalently linked to an oxygen atom. Examples of alkoxy groups or alkoxyl radicals include, but are not limited to, methoxy, ethoxy, isopropyloxy, propoxy, butoxy and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy and trichloromethoxy.

As used herein, the term "aryl," refers to stable aromatic ring system, that may be monocyclic or polycyclic, of which all the ring atoms are carbon, and which may be substituted or unsubstituted. The aromatic ring system may have, for example, 3-7 ring atoms. Examples include phenyl, benzyl, naphthyl, anthracyl, and the like. Each instance of an aryl group may be independently optionally substituted, i.e., unsubstituted (an "unsubstituted aryl") or substituted (a "substituted aryl") with one or more substituents.

As used herein, the term "heteroaryl" refers to an aryl group that includes one or more ring heteroatoms. For example, a heteroaryl can include a stable 5-, 6-, or 7-membered monocyclic or 7-, 8-, or 9-membered bicyclic aromatic heterocyclic ring which consists of carbon atoms and one or more heteroatoms, independently selected from the group consisting of nitrogen, oxygen and sulfur. The nitrogen atom may be substituted or unsubstituted (e.g., N or NR₄ wherein R₄ is H or other substituents, as defined). Examples of heteroaryl groups include pyrrole, furan, indole, thiophene, thiazole, isothiazole, imidazole, triazole, tetrazole, pyrazole, oxazole, isoxazole, pyridine, pyrazine, pyridazine, pyrimidine, and the like.

As used herein, the terms "cycloalkylene," "heterocyclylene," "arylene," and "heteroarylene," alone or as part of another substituent, mean a divalent radical derived from a cycloalkyl, heterocyclyl, aryl, and heteroaryl, respectively. Each instance of a cycloalkylene, heterocyclylene, arylene, or heteroarylene may be independently optionally substituted, i.e., unsubstituted (an "unsubstituted arylene") or substituted (a "substituted heteroarylene") with one or more substituents.

As used herein, the term "cycloalkyl" is intended to include non-aromatic cyclic hydrocarbon rings, such as hydrocarbon rings having from three to eight carbon atoms in their ring structure. Cycloalkyl can include cyclobutyl, cyclopropyl, cyclopentyl, cyclohexyl and the like. The cycloalkyl group can be either monocyclic ("monocyclic cycloalkyl") or contain a fused, bridged or spiro ring system such as a bicyclic system ("bicyclic cycloalkyl") and can be saturated or can be partially unsaturated. "Cycloalkyl" also includes ring systems wherein the cycloalkyl ring, as defined above, is fused with one or more aryl groups wherein the point of attachment is on the cycloalkyl ring, and in such instances, the number of carbons continue to designate the number of carbons in the cycloalkyl ring system. Each instance of a cycloalkyl group may be independently optionally substituted, i.e., unsubstituted (an "unsubstituted cycloalkyl") or substituted (a "substituted cycloalkyl") with one or more substituents.

As used herein, the term "heterocyclyl" refers to a monovalent cyclic molecular structure comprising atoms of at least two different elements in the ring or rings (i.e., a radical of a heterocyclic ring). Additional reference is made to: Oxford Dictionary of Biochemistry and Molecular Biology, Oxford University Press, Oxford, 1997 as evidence that heterocyclic ring is a term well-established in field of organic chemistry.

As used herein, the term "dipeptide" refers to a peptide that is composed of two amino acid residues, which may be denoted herein as -A₁-A₂-. For example, dipeptides employed in the synthesis of a protease-cleavable linker-payload conjugate of the present disclosure may be selected from the group consisting of Val-Cit, Phe-Lys, Trp-Lys, Asp-Lys, Val-Lys, and Val-Ala.

As used herein, the term "halo" or "halogen" refers to F, Cl, Br, or I.

The aryl or heteroaryl aromatic ring can be substituted at one or more ring positions with such substituents as described above, for example, alkyl, alkenyl, akynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

As used herein, the term "hydroxy" refers to a group of formula -OH.

As used herein, the term "hydroxyl" refers to a hydroxyl radical (.OH).

As used herein, the term "oxo" refers to an oxygen that is double bonded to carbon or another element (i.e., =O).

As used herein, the phrase "optionally substituted" means unsubstituted or substituted. In general, the term "substituted" means that at least one hydrogen present on a group (e.g., a carbon or nitrogen atom) is replaced with a permissible substituent, e.g., a substituent which upon substitution results in a stable compound. The term "substituted" can include substitution with all permissible substituents of organic compounds, such as any of the substituents described herein that result in the formation of a stable compound. For purposes of the present disclosure, heteroatoms such as nitrogen may have hydrogen substituents and/or any suitable substituent as described herein which satisfy the valencies of the heteroatoms and results in the formation of a stable moiety.

As used herein, a "targeting ligand" is a molecule that can be bonded to a nanoparticle and target the nanoparticle to a tumor or cancer cell, typically by binding to the tumor or cancer cell (such as by binding to a protein expressed on the surface of the tumor or cancer cell). The targeting ligand can be any suitable molecule such as a small organic molecule (e.g., folate or a folate analog), an antigen-binding portion of an antibody (e.g. a Fab fragment, a Fab' fragment, a F(ab')2 fragment, a scFv fragment, a Fv fragment, a dsFv diabody, a dAb fragment, a Fd' fragment, a Fd fragment, or an isolated complementarity determining region (CDR) region), an antibody mimetic (e.g., an aptamer, affibody, affilin, affimer, anticalin, avimer, Darpin, and the like), the binding domain of a receptor, a nucleic acid, lipid and the like.

Furthermore, it will be appreciated by one of ordinary skill in the art that the synthetic methods, as described herein, utilize a variety of protecting groups. As used herein, the term "protecting group" refers to a particular functional moiety, e.g., O, S, or N, that is temporarily blocked so that a reaction can be carried out selectively at another reactive site in a multifunctional compound. Protecting groups may be introduced and removed at appropriate stages during the synthesis of a compound using methods that are known to one of ordinary skill in the art. The protecting groups are applied according to standard methods of organic synthesis as described in the literature (Theodora W. Greene and Peter G. M. Wuts (2007) Protecting Groups in Organic Synthesis, 4th edition, John Wiley and Sons, incorporated by reference with respect to protecting groups).

Exemplary protecting groups include, but are not limited to, oxygen, sulfur, nitrogen and carbon protecting groups. For example, oxygen protecting groups include, but are not limited to, methyl ethers, substituted methyl ethers (e.g., MOM (methoxymethyl ether), MTM (methylthiomethyl ether), BOM (benzyloxymethyl ether), PMBM (pimethoxybenzyloxymethyl ether), optionally substituted ethyl ethers, optionally substituted benzyl ethers, silyl ethers (e.g., TMS (trimethylsilyl ether), TES (triethylsilylether), TIPS (triisopropylsilyl ether), TBDMS (t-butyldimethylsilyl ether), tribenzyl silyl ether, TBDPS (t-butyldiphenyl silyl ether), esters (e.g., formate, acetate, benzoate (Bz), trifluoroacetate, dichloroacetate) carbonates, cyclic acetals and ketals. In addition, nitrogen protecting groups include, but are not limited to, carbamates (including methyl, ethyl and substituted ethyl carbamates (e.g., Troc), amides, cyclic imide derivatives, N-Alkyl and N-Aryl amines, imine derivatives, and enamine derivatives, etc. Amino protecting groups include, but are not limited to fluorenylmethyloxycarbonyl (Fmoc), tert-butyloxycarbonyl (Boc), carboxybenzyl (Cbz), acetamide, trifluoroacetamide, etc. Certain other exemplary protecting groups are detailed herein, however, it will be appreciated that the present disclosure is not intended to be limited to these protecting groups; rather, a variety of additional equivalent protecting groups may be utilized according to methods known to one skilled in the art.

Throughout this disclosure, a nanoparticle drug conjugate (NDC) may sometimes be referred to as a CDC (C'Dot-drug-conjugate), e.g., a FA-CDC, or simply as a functionalized nanoparticle.

### Nanoparticles

The methods disclosed herein may be used to functionalize any suitable nanoparticle. For example, silica nanoparticles can be functionalized using a method disclosed herein (including nanoparticles that are a partially comprised of silica, or completely comprised of silica). The nanoparticle can have a diameter from about 0.5 nm to about 100 nm, *e.g.,* from about 0.1 nm to about 50 nm, from about 0.5 nm to about 25 nm, from about 1 nm to about 20 nm, from about 0.8 nm to about 15 nm, from about 1 nm to about 10 nm, or from about 1 nm to about 8 nm, e.g., about 1 nm, about 2 nm, about 3 nm, about 4 nm, about 5 nm, about 6 nm, about 7 nm, about 8 nm, about 9 nm, about 10 nm, about 11 nm, or about 12 nm.

The nanoparticle may comprise a core surrounded by a shell (*i.e.,* a core-shell nanoparticle). Alternatively, the nanoparticle may have only a core and no shell. The nanoparticle may also comprise a material (*e.g.*, on its surface) that is reactive with a silane moiety, *e.g.,* a silica surface. The shell of the nanomaterial may be a material that is reactive with a silane moiety, *e.g.,* a silica shell. The methods disclosed herein can be used to modify a silica nanoparticle that comprises a silica-based core and a silica-based shell surrounding at least a portion of the core (a core-shell silica nanoparticle). The nanoparticle may be a non-mesoporous nanoparticle (*e.g.,* a non-mesoporous core-shell nanoparticle), such as a non-mesoporous silica nanoparticle (*e.g.,* a non-mesoporous core-shell silica nanoparticle).

The nanoparticle may comprise an organic polymer coating on its surface. An organic polymer that may be attached to the nanoparticle includes, but is not limited to, polyethylene glycol) (PEG), polylactate, polylactic acids, sugars, lipids, polyglutamic acid (PGA), polyglycolic acid, poly(lactic-co-glycolic acid) (PLGA), polyvinyl acetate (PVA), and combinations thereof. For example, the nanoparticle may have a layer of polyethylene glycol (PEG) molecules attached to the surface. Certain organic polymer coatings can provide a nanoparticle with advantageous properties, such as properties suitable for a biological system. For example, following administration of a nanoparticle comprising a PEG coating to an animal (e.g., human), the PEG groups may prevent adsorption of serum proteins to the nanoparticle and may facilitate efficient urinary excretion, and may decrease aggregation of the nanoparticle (*see, e.g.,* Burns et al. "Fluorescent silica nanoparticles with efficient urinary excretion for nanomedicine", Nano Letters (2009) 9(1):442-448, which is incorporated herein by reference in its entirety).

The shell of the nanoparticle may have a range of layers. For example, the shell may comprise from about 1 to about 20 layers, from about 1 to about 15 layers, from about 1 to about 10 layers, or from about 1 to about 5 layers. For example, the shell may comprise from about 1 to about 3 layers. The thickness of the shell may range from about 0.5 nm to about 90 nm, *e.g.,* from about 1 nm to about 40 nm, from about 1 nm to about 20 nm, from about 1 nm to about 10 nm, or from about 1 nm to about 5 nm. For example, the thickness of the shell may be from about 1 nm to about 2 nm. The shell of the nanoparticle may cover only a portion of nanoparticle or the entire particle. For example, the shell may cover about 1 to about 100 percent, from about 10 to about 80 percent, from about 20 to about 60 percent, or from about 30 to about 50 percent of the nanoparticle. The shell of the nanoparticle can comprise silica, and/or may be the reaction product of a silica forming compound, such as a tetraalkyl orthosilicate, for example tetraethyl orthosilicate (TEOS).

The nanoparticle (e.g., the nanoparticle core and/or shell) can be substantially non-porous, meso-porous, semi-porous, or porous. The nanoparticle may comprise a non-pore surface and a pore surface. The pore surface may be referred to as the interior surface. The nanoparticle may also have a non-pore surface (or non-porous surface). The non-pore surface may be referred to herein as the exterior nanoparticle surface. The pore surface (e.g., at least a portion of the pore surface) and/or the non-pore surface (e.g., at least a portion of the non-pore surface) of the nanoparticle can be functionalized. As stated above, the nanoparticle and/or nanoparticle shell may be non-mesoporous.

The nanoparticle may contain a dye, such as a fluorescent compound. The dye can be contained within the nanoparticle core. For example, the nanoparticle may contain a dye covalently encapsulated in the nanoparticle. The dye in a silica nanoparticle may be the reaction product of a reactive dye (e.g., fluorescent compound) and a co-reactive organo-silane compound. A silica nanoparticle may contain the reaction product of a reactive dye compound and a co-reactive organo-silane compound, and silica.

The nanoparticles may incorporate any known fluorescent compound, such as fluorescent organic compound, dyes, pigments, or combinations thereof. Such fluorescent compounds can be incorporated into the silica matrix of the core of a silica nanoparticle. A wide variety of suitable chemically reactive fluorescent dyes/fluorophores are known, see for example, Molecular Probes Handbook of Fluorescent Probes and Research Chemicals, 6th ed., R. P. Haugland, ed. (1996). The fluorescent compound may be covalently encapsulated within the core of the nanoparticle.

The fluorescent compound can be, but is not limited to, a near infrared fluorescent (NIRF) dye that is positioned within the core of the nanoparticle, that can provide greater brightness and fluorescent quantum yield relative to the free fluorescent dye. It is well-known that the near infrared-emitting probes exhibit decreased tissue attenuation and autofluorescence (Burns et al. *supra*)*.* Fluorescent compounds that may be used (e.g., encapsulated by an NDC) in the present disclosure, include, but are not limited to, Cy5, Cy5.5 (also known as Cy5++), Cy2, fluorescein isothiocyanate (FITC), tetramethylrhodamine isothiocyanate (TRITC), phycoerythrin, Cy7, fluorescein (FAM), Cy3, Cy3.5 (also known as Cy3++), Texas Red (sulforhodamine 101 acid chloride), LIGHTCYCLER^{®}-Red 640, LIGHTCYCLER^{®}-Red 705, tetramethylrhodamine (TMR), rhodamine, rhodamine derivative (ROX), hexachlorofluorescein (HEX), rhodamine 6G (R6G), the rhodamine derivative JA133, Alexa Fluorescent Dyes (such as ALEXA FLUOR^{®} 488, ALEXA FLUOR^{®} 546, ALEXA FLUOR^{®} 633, ALEXA FLUOR^{®} 555, and ALEXA FLUOR^{®} 647), 4',6-diamidino-2-phenylindole (DAPI), propidium iodide, aminomethylcoumarin (AMCA), Spectrum Green, Spectrum Orange, Spectrum Aqua, LISSAMINE^{™}, and fluorescent transition metal complexes, such as europium. Fluorescent compounds that can be used also include fluorescent proteins, such as GFP (green fluorescent protein), enhanced GFP (EGFP), blue fluorescent protein and derivatives (BFP, EBFP, EBFP2, azurite, mKalama1), cyan fluorescent protein and derivatives (CFP, ECFP, Cerulean, CyPet) and yellow fluorescent protein and derivatives (YFP, Citrine, Venus, YPet) (WO 2008/142571, WO 2009/056282, WO 1999/22026). In preferred aspects, the fluorescent compound is Cy5.

The nanoparticle may be synthesized by the steps of: (1) covalently conjugating a fluorescent compound, such as a reactive fluorescent dye (e.g., Cy5), with a reactive moiety including, but not limited to, maleimide, iodoacetamide, thiosulfate, amine, N-hydroxysuccimide ester, 4-sulfo-2,3,5,6-tetrafluorophenyl (STP) ester, sulfosuccinimidyl ester, sulfodichlorophenol esters, sulfonyl chloride, hydroxyl, isothiocyanate, carboxyl, to an organo-silane compound, such as a co-reactive organo-silane compound, to form a fluorescent silica precursor, and reacting the fluorescent silica precursor to form a fluorescent core; or (2) reacting the fluorescent silica precursor with a silica forming compound, such as tetraalkoxysilane, to form a fluorescent core and reacting the resulting core with a silica forming compound, such as a tetraalkoxysilane, to form a silica shell on the core, to provide the fluorescent nanoparticle.

Fluorescent silica-based nanoparticles are known in the art and are described in U.S. Patent Nos. 8,298,677; 9,625,456; 10,548,997; 9,999,694; 10,039,847; and 10,548,998; the contents of which are each incorporated herein by reference in their entireties.

The nanoparticles functionalized by methods disclosed herein may comprise a nanoparticle that comprises a silica-based core and a silica shell surrounding at least a portion of the core, and polyethylene glycol (PEG) covalently bonded to the surface of the nanoparticle. The nanoparticles may have a fluorescent compound covalently encapsulated within the core of the nanoparticle. For example, ultrasmall PEGylated silica nanoparticles, referred to as C'Dots, can be functionalized using the methods disclosed herein. C'dots can be prepared as described previously (see, e.g., Ma, K. et al. Chemistry of Materials (2015) 27(11):4119-4133; the contents of which are incorporated herein by reference in its entirety). Nanoparticles for use in a method described herein may also be prepared as described in Ma, K. et al. Chem. Mater. (2013), 25:677-691; Ma, K. et al. Chem. Mater. (2016) 28:1537-1545; Ma, K. et al. Chem. Mater. (2017) 29:6840-6855; WO 2016/179260; and WO 2018/213851. Each C'Dot comprises a PEGylated silica particle (approximately 6 nm in diameter) in which near-infrared fluorescent Cy5 dyes are covalently encapsulated.

### Methods of Functionalizing Nanoparticles

Using the methods disclosed herein, a nanoparticle can be modified to incorporate one or more functional groups on the surface of the nanoparticle, which may be further modified (*e.g.,* by conjugating the functional group to a molecule). For example, a material on the surface of the nanoparticle that is reactive with a silane (e.g., a silanol group) can be reacted with a bifunctional precursor disclosed herein, such as a bifunctional precursor comprising a silane group and another functional group, to attach the another functional group to the nanoparticle surface. The material reactive with the silane group may be underneath an organic polymer layer coating of the nanoparticle (e.g., underneath a PEG layer), and the bifunctional precursor may insert into the interstitial space of the organic polymer molecules, and react with the material on the nanoparticle surface.

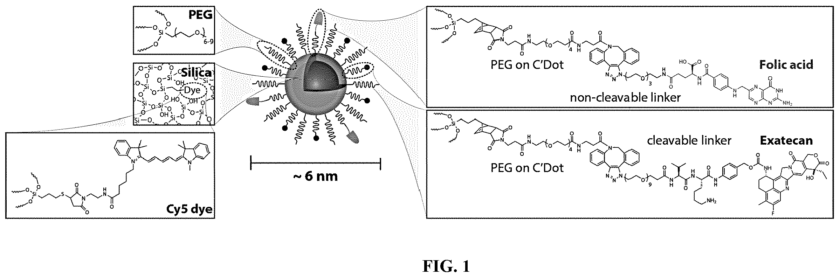

Using the methods disclosed herein, a nanoparticle can be functionalized with one or more alkyne moieties, which can be further conjugated with a compound comprising an alkyne-reactive group (e.g., azide, diene, nitrone, or nitrile oxide). As such, the methods disclosed herein can permit functionalization of a nanoparticle for a particular use, *e.g.,* for use in targeted cancer therapy. For example, the compound comprising an alkyne-reactive group may also comprise a moiety that can target (*e.g.,* has an affinity for) a particular biological target (*e.g.,* a cancer cell, e.g., a receptor expressed by a cancer cell). For example, the molecule may be a targeting ligand, such as a compound that binds to a certain receptor, e.g., a compound that binds to a folate receptor, such as folic acid or a derivative thereof. As such, the method disclosed herein can be used to functionalize nanoparticles that are suitable for targeting cancer cells. The compound comprising an alkyne-reactive group may be a cytotoxic compound, and can include a cleavable linker, such that the cytotoxic compound can be released in a biological system (*e.g.,* within a cancer cell) upon cleavage of the linker. The payload can be a cytotoxic drug, such as a small molecule chemotherapeutic drug, *e.g.,* exatecan or a derivative thereof, and the linker can be a protease-cleavable linker. For example, an exemplary nanoparticle drug conjugate (NDC) comprising an exatecan payload and a folic acid targeting ligand, made using a method disclosed herein, is depicted in FIG. 1.

The methods disclosed herein can be used to functionalize a nanoparticle that is coated with an organic polymer. For example, the method described herein may be carried out after the nanoparticle has been coated with polyethylene glycol (post-PEGylation). This reduces the need for reaction optimization, and minimizes alterations needed in both the synthetic protocol and to the nanoparticle itself (e.g., no alterations are needed to optimize surface chemistry of the nanoparticle prior to carrying out a method disclosed herein), which is more efficient than conventional methods for nanoparticle functionalization. Using conventional nanoparticle functionalization approaches, it is usually not possible or is highly inefficient to introduce a functional group to a nanoparticle that has an organic polymer coating (e.g., PEG), due in part to the steric hindrance of the organic polymer that prevents conj ugating precursor molecules to the nanoparticle surface, and these conventional methods typically provide low yields or fail to provide any functionalized nanoparticle. The typical way to avoid these issues for functionalizing nanoparticles is to introduce precursor molecules (containing desired functional groups) to the surface of the nanoparticle simultaneously with the addition of an organic polymer coating molecule. However, this approach has significant shortcomings, such as unpredictable reaction kinetics (e.g., due to attraction or repulsion between functional groups of the precursor molecules and the nanoparticle surface), functional groups becoming 'buried' in the organic polymer layer of the nanoparticle during the synthesis, and aggregation of partially coated nanoparticles during synthesis, each of which result in low yields and sub-optimal functionalization of the nanoparticle. This is a particular issue when using silica nanoparticles that possess a charged surface, which can exacerbate undesired intermolecular interactions and affect reaction kinetics in unpredictable ways. The methods disclosed herein are able to overcome these problems.

Additionally, the methods disclosed herein provide a solution to the problem of how to attach bulky molecules, such as DBCO, to the surface of a nanoparticle that is sterically hindered by an organic polymer coating (e.g., PEG layer). Certain molecules, particularly those having larger size and steric hindrance, have limited access the surface of the nanoparticle, resulting in lower reactivity and hindering attachment of the molecules to the nanoparticle surface. For example, only two DBCO groups can be attached to a nanoparticle when using DBCO-PEG-silane as a bifunctional precursor, even when the DBCO-PEG-silane precursor is at a relatively high ratio in the reaction mixture (see, e.g., Example 1 and FIG. 2).

The methods disclosed can involve a two-step reaction sequence, where a small first bifunctional precursor (e.g., a precursor comprising a silane group) that has a relatively high diffusion coefficient is first bonded to the nanoparticle surface, and is then subsequently modified by another compound, e.g., an additional bifunctional precursor(s). For example, a small bifunctional precursor comprising a silane moiety and another reactive group (e.g., a diene, an amine, a thiol, a hydroxyl, an azide, an alkene, a nitrone, a nitrile oxide, or an alkyne) can be added to a solution of a nanoparticle comprising a surface that is reactive with the silane group, under conditions suitable for reaction between the nanoparticle surface and the silane group, thereby providing a nanoparticle that is functionalized with the another reactive group.

The conditions suitable for reaction between the silane moiety and the nanoparticle surface may comprise combining the nanoparticle and the first bifunctional precursor in an aqueous reaction medium (e.g., a reaction medium that is substantially water). The reaction medium may also comprise an aprotic organic solvent, such as dimethylsulfoxide (DMSO) or acetonitrile). For example, the reaction medium may comprise no more than 20 v/v% of the aprotic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the aprotic solvent. The reaction medium may also comprise a protic organic solvent, such as an alcohol (e.g., tert-butanol). For example, the reaction medium may comprise no more than 20 v/v% of the protic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the protic solvent. The reaction medium may comprise a buffer (e.g., an aqueous buffer), such as phosphate buffered saline (PBS). For example, the reaction medium may comprise a buffer (e.g., an aqueous buffer, such as PBS) at a final concentration between about 1× and about 5x, e.g., about 1x, about 2x, about 3x, about 4x, or about 5x. The reaction medium may be substantially free of an organic solvent (e.g., substantially free of organic protic solvent, substantially free of aprotic solvent, or substantially free of any organic solvent). The reaction medium may be substantially free of buffer. The reaction medium may be substantially water.

The conditions suitable for reaction between the silane moiety and the nanoparticle surface may comprise combining the nanoparticle and the first bifunctional precursor in a reaction medium for a certain period of time, e.g., between about 1 minute and about 60 minutes, between about 0.5 hours and about 24 hours, between about 0.5 hours and about 18 hours, between about 0.5 hours and about 12 hours, between about 0.5 hours and about 6 hours, between about 0.5 hours and about 4 hours, between about 0.5 hours and about 3 hours, between about 0.5 hours and about 2 hours, between about 0.5 hours and about 1 hours, or between about 1 hour and about 48 hours. For example, the conditions may comprise holding the reaction medium overnight.

The conditions suitable for reaction between the silane moiety and the nanoparticle surface may comprise combining the nanoparticle and the first bifunctional precursor in a reaction medium, and heating the reaction medium, e.g., to a temperature of about 20 °C or greater, e.g., about 35 °C or greater, about 40 °C or greater, about 45 °C or greater, about 50 °C or greater, about 60 °C or greater, about 70 °C or greater, about 80 °C or greater, about 90 °C or greater, about 100 °C or greater, about 110 °C or greater, or about 120 °C or greater, e.g., between about 20 °C and about 60 °C, between about 40 °C and about 80 °C, between about 60 °C and about 100 °C, between about 20 °C and about 40 °C, between about 30 °C and about 50 °C, between about 40 °C and about 60 °C, between about 50 °C and about 70 °C, between about 60 °C and about 80 °C, between about 70 °C and about 90 °C, between about 80 °C and about 100 °C, or between about 90 °C and about 110 °C. The conditions may comprise maintaining the nanoparticle and the first bifunctional precursor at room temperature. The conditions suitable for a reaction between the silane moiety and the nanoparticle surface may comprise stirring, shaking, or applying other mixing methods to the reaction medium.

The first bifunctional precursor can be any suitable silane-containing compound that is reactive with the nanoparticle surface, and may comprise a functional group that will be available for further modification, e.g., after attachment of the first bifunctional precursor to the nanoparticle. The functional group may be a group that is not reactive with the nanoparticle, and/or not reactive with a silane. The first bifunctional precursor may comprise an alkylene group (e.g., a C₁-C₆ alkylene group), with a silane group at one terminus, and another functional group at the other terminus (e.g., a diene, an amine, a thiol, a hydroxyl, an azide, an alkene, a nitrone, a nitrile oxide, or an alkyne). The first bifunctional precursor may comprise a heteroalkylene group (e.g., a C₁-C₆ heteroalkylene group), with a silane group at one terminus, and another functional group at the other terminus (e.g., a diene, an amine, a thiol, a hydroxyl, an azide, an alkene, a nitrone, a nitrile oxide, or an alkyne). The first bifunctional precursor can comprise an alkylene group (e.g., a C₁-C₆ alkylene group), with a silane group at one terminus, and a diene group at the other terminus, such as a cyclic dienyl group (e.g., a cyclopentadienyl group). Any suitable diene group may be used. For example, any diene suitable to undergo a Diels-Alder (or Hetero-Diels Alder) cycloaddition may be used. Examples of dienes that may be present in the first bifunctional precursor include, but are not limited to, cyclopentadiene, cyclohexadiene, furan, butadiene, and derivatives thereof. Also, other moieties capable of modification, e.g., by a reaction with another entity, may be present on the first bifunctional precursor, and the method is not limited to using Diels-Alder cycloadditions (e.g., other cycloadditions, or other bond-forming reactions, can be used, such as another bond-forming reaction described herein).

The first bifunctional precursor can comprise a structure of Formula (A): 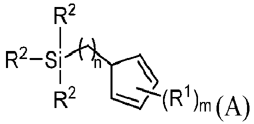 wherein R¹ is alkyl, alkenyl, alkynyl, heteroalkyl, cycloalkyl, heterocyclyl, aryl, heteroaryl, halo, -OR^{A}, -NR^{B}R^{C}, -NO₂, or -CN, wherein each alkyl, alkenyl, alkynyl, heteroalkyl, cycloalkyl, heterocyclyl, aryl, or heteroaryl, may be substituted or unsubstituted; each R² is independently hydrogen, alkyl, halo, or -OR^{A}; R^{A}, R^{B}, and R^{C} are each independently hydrogen, alkyl, alkenyl, alkynyl, heteroalkyl, cycloalkyl, heterocyclyl, aryl, or heteroaryl; n is an integer of 0 to 12; and m is an integer of 0 to 5. For example, R¹ can be alkyl, C3-alkenyl, C3-alkynyl, heteroalkyl, cycloalkyl, heterocyclyl, aryl, heteroaryl, halo, -OR^{A}, wherein each alkyl, C3-alkenyl, C3-alkynyl, heteroalkyl, cycloalkyl, heterocyclyl, aryl, or heteroaryl, may be substituted or unsubstituted; each R² is independently hydrogen, alkyl, halo, or -OR^{A}; R^{A} is hydrogen, alkyl, alkenyl, alkynyl, heteroalkyl, cycloalkyl, heterocyclyl, aryl, or heteroaryl; n is an integer of 0 to 12; and m is an integer of 0 to 5. For example, R² may be -OR^{A}, wherein R^{A} is alkyl (e.g., methyl or ethyl); n may be an integer of 1 to 5 (e.g., 3); and m may be 0. In some aspects, R² is - OEt; n is 3; and m is 0. For example, the first bifunctional precursor can comprise the structure of Formula (A-1):

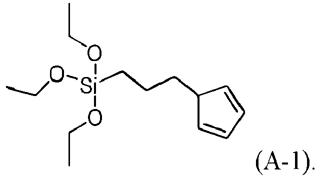

The first bifunctional precursor may be (3-cyclopentadienylpropyl)triethoxysilane (e.g., as demonstrated in Example 3), wherein the method provides a nanoparticle functionalized with one or more cyclopentadienyl groups. Alternatively, the first bifunctional precursor may be (3-aminopropyl)triethoxysilane (e.g., as demonstrated in Example 2), wherein the method provides a nanoparticle functionalized with one or more amine groups.

The step of reacting the nanoparticle with the first bifunctional precursor can provide a nanoparticle functionalized with a first reactive group, which may be further modified. For example, the nanoparticle functionalized with a first reactive group may be contacted with a second bifunctional precursor that comprises a moiety reactive with the first reactive group.

Contacting the nanoparticle functionalized with a first reactive group with the second bifunctional precursor can be under any conditions suitable for a reaction between first reactive group and the moiety reactive with the first reactive group. For example, the conditions suitable for a reaction between first reactive group and the moiety reactive with the first reactive group can comprise an aqueous reaction medium (e.g., a reaction medium that is substantially water). The reaction medium may comprise a buffer (e.g., an aqueous buffer), such as phosphate buffered saline (PBS). For example, the reaction medium may comprise a buffer (e.g., an aqueous buffer, such as PBS) at a final concentration between about 1× and about 5x, e.g., about 1×, about 2x, about 3x, about 4x, or about 5x. The reaction medium may also comprise an aprotic organic solvent, such as dimethylsulfoxide (DMSO) or acetonitrile. For example, the reaction medium may comprise no more than 20 v/v% of the aprotic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the aprotic solvent. The reaction medium may also comprise a protic organic solvent, such as an alcohol (e.g., tert-butanol). For example, the reaction medium may comprise no more than 20 v/v% of the protic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the protic solvent. The reaction medium may be substantially free of an organic solvent (e.g., substantially free of organic protic solvent, substantially free of aprotic solvent, or substantially free of any organic solvent). The reaction medium may be substantially water.

The conditions suitable for a reaction between first reactive group and the moiety reactive with the first reactive group can comprise holding the reaction mixture for a certain period of time, e.g., between about 1 minute and about 60 minutes, between about 0.5 hours and about 24 hours, between about 0.5 hours and about 18 hours, between about 0.5 hours and about 12 hours, between about 0.5 hours and about 6 hours, between about 0.5 hours and about 4 hours, between about 0.5 hours and about 3 hours, between about 0.5 hours and about 2 hours, between about 0.5 hours and about 1 hours, or between about 1 hour and about 48 hours. For example, the reaction medium may held overnight.

The conditions suitable for a reaction between first reactive group and the moiety reactive with the first reactive group can comprise heating the reaction medium, e.g., to a temperature of about 20 °C or greater, e.g., about 35 °C or greater, about 40 °C or greater, about 45 °C or greater, about 50 °C or greater, about 60 °C or greater, about 70 °C or greater, about 80 °C or greater, about 90 °C or greater, about 100 °C or greater, about 110 °C or greater, or about 120 °C or greater, e.g., between about 20 °C and about 60 °C, between about 40 °C and about 80 °C, between about 60 °C and about 100 °C, between about 20 °C and about 40 °C, between about 30 °C and about 50 °C, between about 40 °C and about 60 °C, between about 50 °C and about 70 °C, between about 60 °C and about 80 °C, between about 70 °C and about 90 °C, between about 80 °C and about 100 °C, or between about 90 °C and about 110 °C. The conditions may also comprise maintaining the reaction mixture at room temperature. The conditions suitable for a reaction between first reactive group and the moiety reactive with the first reactive group can also comprise stirring, shaking, or applying other mixing methods to the reaction medium.

The first reactive group can be a diene (e.g., cyclopentadiene), and the second bifunctional precursor can comprise a dienophile (e.g., a dienophile described herein, e.g., maleimide) that is reactive with the first reactive group, wherein the contacting may be under conditions sufficient for promoting a Diels-Alder cycloaddition between a diene and dienophile.

The dienophile can be any suitable dienophile, such as a moiety comprising an electron deficient alkene. The dienophile may comprise a cyclic dienophile, e.g., a maleimide, a quinone, a maleic anhydride, dialkyl acetylene dicarboxylic acid, or any derivative thereof.

It will be understood that the position of the diene and dienophile can be on either the first bifunctional precursor or second bifunctional precursor, respectively. In other words, while certain methods exemplified herein feature a first bifunctional precursor comprising a diene, and a second bifunctional precursor comprising a dienophile, these methods or precursors can be readily modified such that the first bifunctional precursor comprises the dienophile, and the second bifunctional precursor comprises the diene. Additionally, while the reaction between dienes and dienophiles has been exemplified in this disclosure, the conjugation between the first reactive group and the second bifunctional precursor can involve any other suitable covalent bond forming reaction, which can be determined by selection of groups on the first bifunctional precursor and second bifunctional precursor. For example, the methods disclosed herein to functionalize a nanoparticle (e.g., at any step of the process) can involve etherifi cation, amide bond formation, Click Chemistry, Diels-Alder cycloaddition, Hetero-Diels-Alder cycloaddition, 1,2-addition such as a Michael addition, Huisgen cycloaddition, nitrone-olefin cycloaddition, 3+2 cycloaddition, 4+2 cycloaddition, olefin metathesis, or any reaction described in Kolb et al. Angew. Chem. Int. Ed. (2001) 40:2004-2021 (incorporated herein by reference in its entirety). For example, instead of using a Diels-Alder reaction to conjugate the second bifunctional precursor to the nanoparticle via the first reactive group, the method can be modified such that first reactive group (introduced by the first bifunctional precursor) can be an amine, and the second bifunctional precursor can comprise an N-hydroxysuccinimide (NHS) ester group, and the second bifunctional precursor and functionalized nanoparticle comprising amine group can be contacted under conditions sufficient for promoting an amine-ester reaction and forming a bond between the first reactive group and the second bifunctional precursor. Alternatively, instead of using a Diels-Alder reaction to conjugate the second bifunctional precursor to the nanoparticle via the first reactive group, a click chemistry reaction could be used e.g., where the first reactive group (introduced by the first bifunctional precursor) is an azide or an alkyne, and the second bifunctional precursor is an alkyne or an azide, and the second bifunctional precursor and functionalized nanoparticle can be contacted under conditions sufficient for promoting a click chemistry reaction between the first reactive group and the second bifunctional precursor.

In particular aspects, the first bifunctional precursor comprises a diene (e.g., cyclopentadiene), and the second bifunctional precursor comprises a dienophile (e.g., maleimide) that can be reacted with the diene in a Diels-Alder reaction, to attach the second bifunctional precursor to the nanoparticle (via the first bifunctional precursor), as demonstrated in Example 3, and also as depicted in Scheme 1.

The second bifunctional precursor may comprise yet another reactive group, e.g., on the terminus of the second bifunctional precursor opposite to the moiety that is reactive with the first reactive group. For example, the second bifunctional precursor can comprise a group at one terminus that is reactive with a diene moiety (e.g., a dienophile, e.g., maleimide), and comprise another group at the other terminus suitable for further modification. The another reactive group (sometimes referred to as functional group) on the second bifunctional precursor may be a moiety that is reactive with an alkyne, azide, diene, nitrone, or nitrile oxide. The reactive group may be a moiety that is suitable for undergoing a click chemistry reaction. For example, the reactive group may comprise an alkyne, an azide, a diene, nitrone, or a nitrile oxide. The alkyne can be a strained alkyne. For example, the reactive group may comprise a dibenzoazacyclooctyne (DBCO) group or a derivative thereof (sometimes referred to as DIBAC). The alkyne may comprise a dibenzocyclooctyne group (DIBO) or derivative thereof. The alkyne may also be a terminal alkyne. Alternatively, the reactive group on the second bifunctional precursor may be any other group suitable for conjugation, e.g., a diene, an amine, a thiol, a hydroxyl, an azide, a nitrone, a nitrile oxide, an alkene, or an alkene.

The second bifunctional precursor can also comprise a divalent linker, for example, an alkylene or heteroalkylene group, wherein the alkylene or heteroalkylene group is unsubstituted or substituted (e.g., alkylene or heteroalkylene substituted with one or more oxo group). The divalent linker can comprise a PEG group, e.g., PEG4. The divalent linker can comprise an amide bond, e.g., one, two, three, or more amide bonds. Each amide bond may be in any orientation. For example, an amide bond may be situated in the divalent linker between a PEG group and an alkylene group, wherein the carbon atom of the carbonyl group of the amide is covalently attached to an atom of the PEG group, and the nitrogen atom of the amide is covalently attached to an atom of the alkylene group; or the carbon atom of the carbonyl group of the amide may be attached to an atom of the alkylene group, and the nitrogen atom of the amide group may be attached to an atom of the PEG group. The divalent linker (e.g., heteroalkylene group) may comprise a dienophile moiety at one terminus, and an alkyne at the other terminus, such as a DBCO group. Alternatively, the second bifunctional precursor can comprise a divalent linker (e.g., heteroalkylene group) with an NHS ester at one terminus, and an alkyne at the other terminus, such as a DBCO group. The divalent linker may comprise a PEG group (e.g., a PEG4 moiety), an amide group, or a combination thereof.

The second bifunctional precursor can comprise a structure of Formula (B) 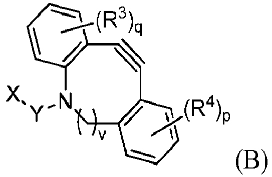 wherein: X is a reactive group, such as a dienophile (e.g., a moiety comprising an electron deficient alkene group; or a cyclic dienophile, e.g., a maleimide, a quinone, or maleic anhydride); Y is a divalent linker (e.g., a substituted or unsubstituted alkylene or heteroalkylene group); R³ and R⁴ are each independently alkyl, alkenyl, alkynyl, heteroalkyl, cycloalkyl, heterocyclyl, aryl, heteroaryl, halo, -OR^{A}, -NR^{B}R^{C}, -NO₂, or -CN, wherein each alkyl, alkenyl, alkynyl, heteroalkyl, cycloalkyl, heterocyclyl, aryl, and heteroaryl may be substituted or unsubstituted; R^{A}, R^{B}, and R^{C} are each independently hydrogen, alkyl, alkenyl, alkynyl, heteroalkyl, cycloalkyl, heterocyclyl, aryl, or heteroaryl; q and p are each independently an integer of 0 to 4; and v is an integer of 0 to 2. For example, X may be maleimide; q and p may each independently be an integer of 0; and v may be an integer of 1.

The divalent linker can comprise a structure of Formula (G): 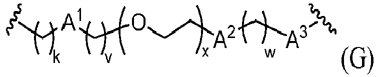 wherein A¹, A², and A³ are each independently selected from the group consisting of alkylene, alkenylene, alkynylene, heteroalkylene, cycloalkylene, arylene, heteroarylene, -C(O)-, -C(O)N(R^{B})-, -N(R^{B})C(O)-, -N(R^{B})-, -OC(O)-, and -C(O)O-; R^{B} is independently hydrogen, alkyl, alkenyl, alkynyl, heteroalkyl, cycloalkyl, heterocyclyl, aryl, or heteroaryl; k, v, w, and x are each independently an integer of 0 to 10; and each 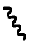 independently denotes a point of attachment to another portion of the second bifunctional precursor. For example, A¹ and A² may each independently be -C(O)N(R^{B})-; A³ may be -C(O)O-; each instance of R^{B} may independently be hydrogen; k, v, and w may each independently be integers of 2; and x may be an integer of 4.

The divalent linker can comprise a structure of Formula (C): 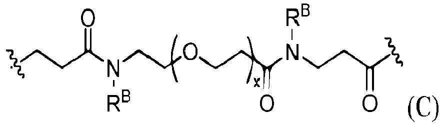 wherein each R^{B} is independently hydrogen, alkyl, alkenyl, alkynyl, heteroalkyl, cycloalkyl, heterocyclyl, aryl, or heteroaryl; x is an integer of 0 to 10; and each \ independently denotes a point of attachment to another portion of the second bifunctional precursor (e.g., an attachment to the dienophile or to the nitrogen atom of the heterocyclic ring of DBCO).

The divalent linker can comprise a structure of Formula (C'): 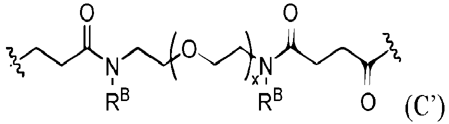 wherein each R^{B} is independently hydrogen, alkyl, alkenyl, alkynyl, heteroalkyl, cycloalkyl, heterocyclyl, aryl, or heteroaryl; x is an integer of 0 to 10; and each 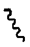 independently denotes a point of attachment to another portion of the second bifunctional precursor (e.g., an attachment to the dienophile or to the nitrogen atom of the heterocyclic ring of DBCO).

The divalent linker can comprise a structure of Formula (C"): 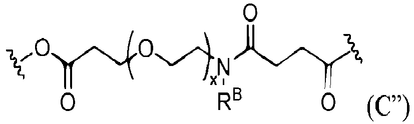 wherein each R^{B} is independently hydrogen, alkyl, alkenyl, alkynyl, heteroalkyl, cycloalkyl, heterocyclyl, aryl, or heteroaryl; x is an integer of 0 to 10; and each \ independently denotes a point of attachment to another portion of the second bifunctional precursor (e.g., an attachment to the nitrogen atom of an NHS moiety, or to the nitrogen atom of the heterocyclic ring of DBCO).

The second bifunctional precursor may comprise a structure of Formula (B-1) 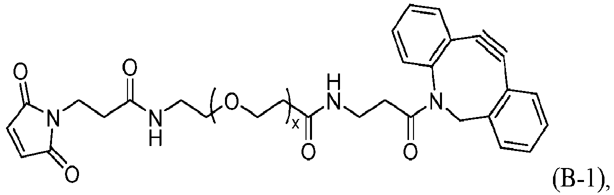 wherein x is an integer of 0 to 10 (e.g., 4). For example, x may be 0, 1, 2, 3, 4, 5, 6, 7, 8, 9, or 10. In preferred embodiments, x is 4.

Reacting a diene moiety (e.g., a diene moiety covalently attached to a nanoparticle surface) with the second bifunctional precursor may provide a compound of Formula (NP-1) 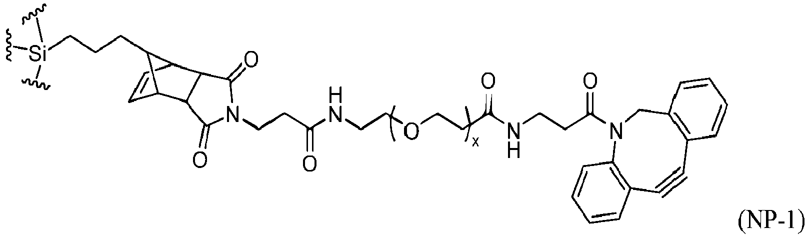 wherein x is an integer of 0 to 10 (e.g., 4), and wherein the silicon atom is a part of a nanoparticle. For example, x may be 0, 1, 2, 3, 4, 5, 6, 7, 8, 9, or 10. In preferred embodiments, x is 4.

The efficiency of the reaction between the second bifunctional precursor (e.g., a DBCO-containing precursor, e.g., DBCO-maleimide) and the functionalized nanoparticle (e.g., nanoparticle comprising a diene moiety) may be independent of the diffusion coefficient of the second bifunctional precursor. For example, second bifunctional precursors disclosed herein that comprise a PEG group of varying length may have similar reactivities with a functionalized nanoparticle, regardless of PEG length (*see e.g.,* Example 4 and FIG. 2, where employing DBCO-PEG-maleimide precursors with varying lengths of PEG did not significantly affect reactivity). This is a departure from typical methods involving other precursors (*e.g.,* precursors shown in Scheme 3 or 4), where the reaction efficiency can be greatly influenced by the size or molecular mass of the precursor. The bifunctional precursors disclosed herein (*e.g.,* second bifunctional precursors) may also have a particular solubility or polarity that can avoid undesired electrostatic interactions (e.g., repulsion) with silanol groups, e.g., silanol groups on another precursor or on a nanoparticle. Additionally, the bifunctional precursors (e.g., second bifunctional precursor) disclosed herein may have a particular length (e.g., on account of a PEG spacer) to endow the resulting functionalized nanoparticle (after conjugation with the second bifunctional precursor) with a desired property, such as improved accessibility for subsequent modification by another precursor, e.g., using click chemistry reactions.

The method disclosed herein may be used to provide nanoparticles suitable for a particular application, such as a therapeutic application. For example, the method can be used to provide nanoparticles suitable for targeting a biological target, e.g., for use in therapy (e.g., cancer therapy), surgical navigation, imaging, diagnostics, or a combination thereof. The nanoparticles may be suitable for *in vivo* use, or can be used *ex vivo* e.g., for analytical or diagnostic applications. The method disclosed herein may be used to make a nanoparticle suitable for targeting a particular receptor (or group of receptors) in a biological system. The method may also be used to prepare a functionalized nanoparticle (e.g., NDC) suitable for delivering a payload to a biological target (or targets). For example, using a method disclosed herein, a nanoparticle can be conjugated with a targeting ligand, and with a payload moiety. For example, the methods disclosed herein can be used to prepare a folate-receptor targeting NDC, e.g., for targeted cancer therapy, such as the exemplary NDC depicted in FIG. 1.

Reacting a nanoparticle with the second bifunctional precursor can provide a nanoparticle comprising one or more groups suitable for conjugation with a molecule (e.g., a targeting ligand and/or payload). For example, reacting the nanoparticle with a second bifunctional precursor comprising an alkyne group can provide a nanoparticle functionalized with one or more alkyne groups, wherein the alkyne groups may be further conjugated with a compound comprising an alkyne-reactive group.

Any desired compound comprising an a reactive group may be attached to a nanoparticle disclosed herein that is functionalized with the appropriate counterpart reactive group. For example, any desired compound comprising an alkyne-reactive group may be attached to the nanoparticle functionalized with an alkyne. For example, a payload moiety (e.g., comprising a cytotoxic drug, e.g., exatecan) or linker-payload conjugate that comprises an alkyne-reactive group may be conjugated to the nanoparticle. Alternatively, a targeting ligand (e.g., a folate receptor (FR)-targeting ligand, e.g., folic acid) that comprises an alkyne reactive group may be conjugated to the nanoparticle. Any combination of compounds may be conjugated to the nanoparticle using this method. For example, both a targeting ligand and a payload moiety can be conjugated to the nanoparticle. Other molecules, such as labels (e.g., radiolabels or dyes), polymers, and/or macromolecules can also be conjugated to a nanoparticle using this method.

For example, a nanoparticle comprising an alkyne moiety (e.g., prepared using a method disclosed herein) can be contacted with a compound comprising an alkyne-reactive group (*e.g.,* an azide, a diene, a nitrone, or a nitrile oxide) under conditions suitable for a reaction between the alkyne moiety and the alkyne-reactive group, thereby forming a nanoparticle functionalized with the compound (e.g., a compound comprising a payload or a targeting ligand).

The conditions suitable for a reaction between the alkyne moiety and the alkyne-reactive group may comprise any suitable click chemistry conditions (*see, e.g.,* Kolb et al. Angew. Chem. Int. Ed. (2001) 40:2004-2021). For example, facilitating a reaction between the alkyne moiety and the alkyne-reactive group may comprise combining the nanoparticle comprising an alkyne moiety and the compound comprising an alkyne-reactive group with a catalyst, such as a copper catalyst (e.g., CuSO₄, CuI, CuCl, Cu(OAc)₂, CuSO₂, CuBr). Other metal catalysts known to effect click chemistry reactions may also be used, such as a ruthenium catalyst. Alternatively, the reaction may be carried out without a catalyst (i.e., catalyst-free conditions, e.g., copper-free conditions). For example, the alkyne on the functionalized nanoparticle may be suitable for catalyst-free click chemistry reactions (e.g., strain-promoted cycloaddition). For example, DBCO can be used in a click chemistry reaction without a catalyst.

The conditions suitable for a reaction between the alkyne moiety and the alkyne-reactive group may comprise combining the nanoparticle comprising an alkyne moiety and the compound comprising an alkyne-reactive group in an aqueous reaction medium (e.g., a reaction medium that is substantially water). The reaction medium may comprise a buffer (e.g., an aqueous buffer), such as phosphate buffered saline (PBS). For example, the reaction medium may comprise a buffer (e.g., an aqueous buffer, such as PBS) at a final concentration between about 1x and about 5x, e.g., about 1×, about 2x, about 3x, about 4x, or about 5x. The reaction medium may also comprise an aprotic organic solvent, such as DMSO or acetonitrile). For example, the reaction medium may comprise no more than 20 v/v% of the aprotic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the aprotic solvent. The reaction medium may also comprise a protic organic solvent, such as an alcohol, e.g., tert-butanol). For example, the reaction medium may comprise no more than 20 v/v% of the protic solvent, e.g., less than 18 v/v%, less than 16 v/v%, less than 14 v/v%, less than 12 v/v%, less than 10 v/v%, less than 8 v/v%, less than 6 v/v%, less than 4 v/v%, less than 2 v/v%, or less than 1 v/v% of the protic solvent. The reaction medium may be substantially free of an organic solvent (e.g., substantially free of organic protic solvent, substantially free of aprotic solvent, or substantially free of any organic solvent). The reaction medium may be substantially water.

The conditions suitable for a reaction between the alkyne moiety and the alkyne-reactive group may comprise combining the nanoparticle comprising an alkyne moiety and the compound comprising an alkyne-reactive group in a reaction medium for a certain period of time, e.g., between about 1 minute and about 60 minutes, between about 0.5 hours and about 24 hours, between about 0.5 hours and about 18 hours, between about 0.5 hours and about 12 hours, between about 0.5 hours and about 6 hours, between about 0.5 hours and about 4 hours, between about 0.5 hours and about 3 hours, between about 0.5 hours and about 2 hours, between about 0.5 hours and about 1 hours, or between about 1 hour and about 48 hours. For example, the reaction medium may be held overnight.

The conditions suitable for a reaction between the alkyne moiety and the alkyne-reactive group may comprise combining the nanoparticle comprising an alkyne moiety and the compound comprising an alkyne-reactive group in a reaction medium, and heating the reaction medium, e.g., to a temperature of about 20 °C or greater, e.g., about 35 °C or greater, about 40 °C or greater, about 45 °C or greater, about 50 °C or greater, about 60 °C or greater, about 70 °C or greater, about 80 °C or greater, about 90 °C or greater, about 100 °C or greater, about 110 °C or greater, or about 120 °C or greater, e.g., between about 20 °C and about 60 °C, between about 40 °C and about 80 °C, between about 60 °C and about 100 °C, between about 20 °C and about 40 °C, between about 30 °C and about 50 °C, between about 40 °C and about 60 °C, between about 50 °C and about 70 °C, between about 60 °C and about 80 °C, between about 70 °C and about 90 °C, between about 80 °C and about 100 °C, or between about 90 °C and about 110 °C. Alternatively, the reaction conditions may comprise maintaining the reaction mixture at room temperature. The conditions suitable for a reaction between first reactive group and the moiety reactive with the first reactive group can also comprise stirring, shaking, or applying other mixing methods to the reaction medium.

The compound comprising a reactive group (e.g., an alkyne-reactive group) may comprise a targeting ligand, and may further comprise a linker (e.g., a non-cleavable linker). The targeting ligand can be a folate receptor targeting ligand. For example, the targeting ligand may be a molecule that can bind to a folate receptor, e.g., a folate receptor expressed on a cancer cell or tumor. The folate-receptor (FR)-targeting ligand may be folic acid, dihydrofolic acid, tetrahydrofolic acid, or a folate-receptor binding derivative thereof. The folate-receptor targeting ligand can be a macromolecule, such as a protein, a peptide, an aptamer, an antibody, or an antibody fragment that can target a folate receptor. For example, the folate-receptor targeting ligand may include a portion of an intact antibody, such as, for example, the antigen-binding or variable region of an antibody. Examples of folate-receptor targeting antibody fragments include, but are not limited to, a Fab fragment, a Fab' fragment, a F(ab')2 fragment, a scFv fragment, a Fv fragment, a dsFv diabody, a dAb fragment, a Fd' fragment, a Fd fragment, or an isolated complementarity determining region (CDR) region. An antigen binding fragment of an antibody may be produced by any means. For example, an antigen binding fragment of an antibody may be enzymatically or chemically produced by fragmentation of an intact antibody and/or it may be recombinantly produced from a gene encoding the partial antibody sequence. Alternatively or additionally, antigen binding fragment of an antibody may be wholly or partially synthetically produced. The linker can be any suitable divalent linker, such as an alkylene or heteroalkylene group. The divalent linker may be a PEG group (e.g., a PEG1, PEG2, PEG3, or PEG4, group).

The compound comprising a reactive group (e.g., an alkyne-reactive group) may comprise a structure of Formula (D'): 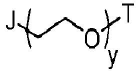 (D'), wherein J is a reactive group, e.g., an alkyne-reactive group; e.g., an azide, a diene, a nitrone, or a nitrile oxide); T is a targeting ligand (e.g., a folate receptor-targeting ligand, e.g., folic acid or a derivative thereof); and y is an integer of 0 to 20 (e.g., 3). For example, y may be 1, 2, 3, 4, 5, 6, 7, 8, 9, 10, 11, 12, 13, 14, 15, 16, 17, 18, 19, or 20, e.g., 2, 3, or 4.

The compound comprising an alkyne-reactive group may comprise a structure of Formula (D): 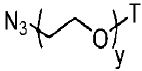 (D), wherein T is a targeting ligand (e.g., a folate receptor-targeting ligand, e.g., folic acid or a derivative thereof); and y is an integer of 0 to 20 (e.g., 3). For example, y may be 1, 2, 3, 4, 5, 6, 7, 8, 9, 10, 11, 12, 13, 14, 15, 16, 17, 18, 19, or 20, e.g., 2, 3, or 4.

The compound comprising a reactive group (e.g., an alkyne reactive group) may comprise a structure of Formula (D-1'): 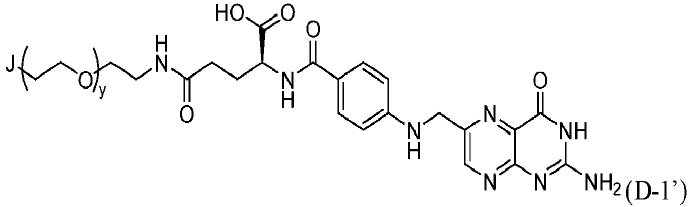 wherein J is a reactive group (e.g., an alkyne-reactive group; e.g., an azide, a diene, a nitrone, or a nitrile oxide); y is an integer of 0 to 20 (e.g., 3). For example, y may be 1, 2, 3, 4, 5, 6, 7, 8, 9, 10, 11, 12, 13, 14, 15, 16, 17, 18, 19, or 20, e.g., 2, 3, or 4.

The compound comprising an alkyne reactive group may comprise a structure of Formula (D-1): 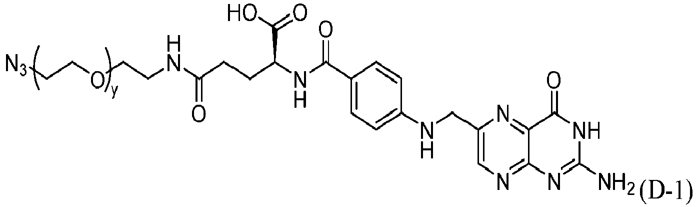 wherein y is an integer of 0 to 20 (e.g., 3). For example, y may be 1, 2, 3, 4, 5, 6, 7, 8, 9, 10, 11, 12, 13, 14, 15, 16, 17, 18, 19, or 20, e.g., 2, 3, or 4.

The compound comprising a reactive group (e.g., an alkyne-reactive group) may comprise a payload moiety, and may further comprise a linker (e.g., a cleavable linker). The payload moiety can be a molecule with a therapeutic use, such as for use in cancer therapy. For example, the payload moiety can be a cytotoxic compound, e.g., a small molecule with cytotoxic properties.

Exemplary payloads include enzyme inhibitors (see e.g., "A Review of Evaluation of Enzyme Inhibitors in Drug Discovery," Robert A. Copeland, John Wiley and Sons, Hoboken, New Jersey), such as topoisomerase inhibitors (e.g., exatecan, SN-38, topotecan, irinotecan, camptothecin, belotecan, indenoisoquinoline, phenanthridines, indolocarbazoles, and analogs thereof), dihydrofolate reductase inhibitors, thymidylate synthase inhibitors, DNA intercalators, DNA minor groove binders, tubulin disruptors, DNA cleavers, anthracyclines, vinca drugs, mitomycins (e.g., mitomycin-C, mitomycin-A), bleomycins, cytotoxic nucleosides, pteridines, diynenes, enediynes, podophyllotoxins, dolastatins, auristatins (e.g., monomethyl auristatin E (MMAE), monomethyl auristatin F (MMAF)), maytansinoids, differentiation inducers, duocarmycin, and taxanes (e.g., taxol). For example, the payload may be exatecan, SN-38, topotecan, irinotecan, belotecan, 9-amino camptothecin, etoposide, camptothecin, taxol, esperamicin, 1,8-dihydroxy-bicyclo[7.3.1]trideca-4-9-diene-2,6-diyne-13-one, podophyllotoxin, anguidine, vincristine, vinblastine, duocarmycin, a pyrrolobenzodiazepine, morpholine-doxorubicin, N-(5,5-diacetoxy-pentyl) doxorubicin, a compound described in U.S. Pat. No. 5,198,560, daunorubicin, doxorubicin, aminopterin, actinomycin, bleomycin, N8-acetyl spermidine, a pyrrolobenzodiazepine, 1-(2 chloroethyl)-1,2-dimethanesulfonyl hydrazide, tallysomycin, cytarabine, a dolastatin, an auristatins, a calicheamicin hydrazide, esperamicin and 6-mercaptopurine, methotrexate, butyric acid, retinoic acid or a derivative thereof.

It will be understood that chemical modifications may be made to the desired payload in order to make reactions of the payload with linker more convenient for purposes of preparing conjugates of the present disclosure. For example a functional group, e.g., amine, hydroxyl, or sulfhydryl, may be appended to the drug at a position which has minimal or an acceptable effect on the activity or other properties of the drug.

The linker may be any suitable divalent linker, and can comprise a portion that is cleavable under certain conditions. For example, the linker can be a self-immolative linker capable of releasing the payload *in vitro* or *in vivo* under certain conditions. The linker may be a protease-cleavable linker (e.g., cleavable by a protease such as cathepsin or trypsin), a pH-sensitive linker, or a redox-sensitive linker. The protease-cleavable linker can be a cathepsin B (Cat-B)-cleavable linker. The linker may comprise a peptide moiety (e.g., a dipeptide), a PEG spacer, and/or a self-immolative moiety (e.g., p-aminobenzyloxycarbonyl (PABC)), e.g., for effective cleavage in a cell, such as a cancer cell. The cleavable linker may be selectively cleaved at a desired location or after a selected time (e.g., upon entry into a targeted cancer cell).

The compound comprising a reactive group (e.g., an alkyne reactive group) may comprise a structure of Formula (E'): 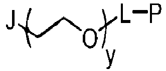 (E'), wherein J is a reactive group (e.g., an alkyne-reactive group; e.g., an azide, a diene, a nitrone, or a nitrile oxide); L is a cleavable linker moiety (e.g., a protease-cleavable linker moiety); P is a payload moiety (e.g., a cytotoxic drug, e.g., exatecan); and y is an integer of 0 to 20 (e.g., 9). For example, L may be a protease-cleavable linker moiety, P may be exatecan, and y may be an integer of 0 to 20 (e.g., 7, 8, 9, 10, or 11).

The compound comprising an alkyne reactive group may comprise a structure of Formula (E): 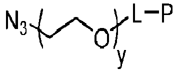 (E), wherein L is a cleavable linker moiety (e.g., a protease-cleavable linker moiety); P is a payload moiety (e.g., a cytotoxic drug, e.g., exatecan); and y is an integer of 0 to 20 (e.g., 9). For example, L may be a protease-cleavable linker moiety, P may be exatecan, and y may be an integer of 0 to 20 (e.g., 7, 8, 9, 10, or 11).

The cleavable linker moiety can comprises a structure of Formula (F): 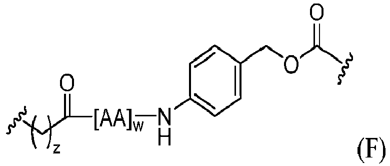 wherein each instance of [AA] is a natural or non-natural amino acid residue; z is an integer of 1 to 5; w is an integer of 1 to 4 (e.g., 2 or 3); and each 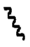 denotes a point of attachment to another portion of the compound comprising a reactive group (e.g., an alkyne-reactive group; e.g., an attachment to a PEG group, an attachment to an alkyne-reactive group, or an attachment to a payload moiety). For example, -[AA]_{w}- may comprise Val-Lys, Val-Cit, Phe-Lys, Trp-Lys, Asp-Lys, Val-Arg, or Val-Ala, and z may be 2, wherein one 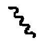 denotes a point of attachment to the oxygen atom of a PEG group, and the other 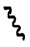 denotes a point of attachment to the nitrogen atom of exatecan.

The cleavable linker moiety can comprises a structure of Formula (F-1). 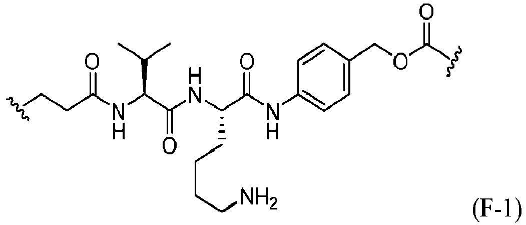 wherein one 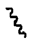 denotes a point of attachment to another portion of the compound comprising a reactive group (e.g., an alkyne-reactive group; e.g., an attachment to a PEG group, an attachment to an alkyne-reactive group, or an attachment to a payload moiety). For example, one 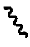 may denote an attachment to the oxygen atom of a PEG group, and the other \ may denote an attachment to the nitrogen atom of exatecan.

The compound comprising a reactive group (e.g., an alkyne reactive group) may comprise a structure of Formula (E-1'): 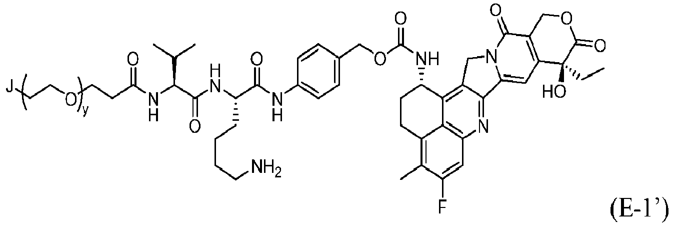 wherein J is a reactive group (e.g., an alkyne-reactive group; e.g., an azide, a diene, a nitrone, or a nitrile oxide); and y is an integer of 0 to 20 (e.g., 9).

The compound comprising an alkyne reactive group may comprise a structure of Formula (E-1): 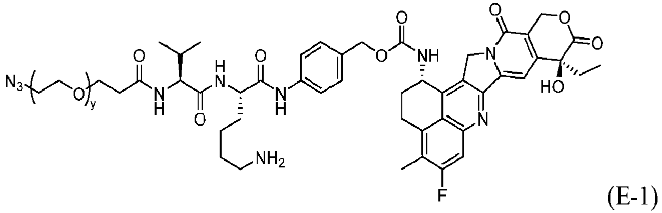 wherein y is an integer of 0 to 20 (e.g., 9).

Reacting an alkyne moiety (e.g., an alkyne covalently attached to a nanoparticle) with a compound comprising an alkyne-reactive group (e.g., a compound of Formula D, e.g., D-1) can provide a compound of Formula (NP-2): 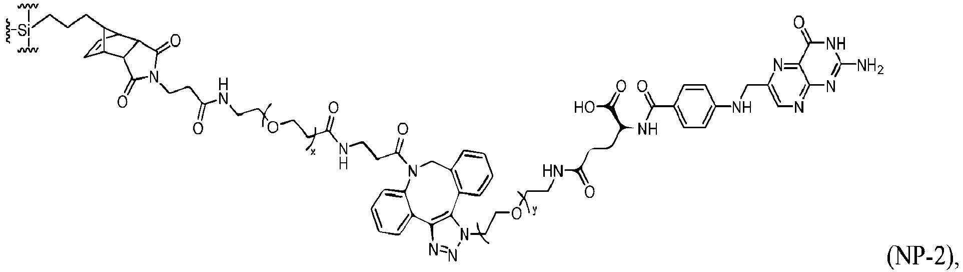 wherein x is an integer of 0 to 10 (e.g., 0, 1, 2, 3, 4, 5, 6, 7, 8, 9, 10, e.g., 4), and y is an integer of 0 to 20 (e.g., 1, 2, 3, 4, 5, 6, 7, 8, 9, 10, 11, 12, 13, 14, 15, 16, 17, 18, 19, or 20, e.g., 3), and the silicon atom is a part of the nanoparticle (e.g., bonded with the silica shell of a core-shell silica nanoparticle).

Reacting an alkyne moiety (e.g., an alkyne moiety covalently attached to a nanoparticle) with a compound comprising an alkyne-reactive group (e.g., a compound of Formula E, e.g., E-1) can provide a compound of Formula (NP-3): 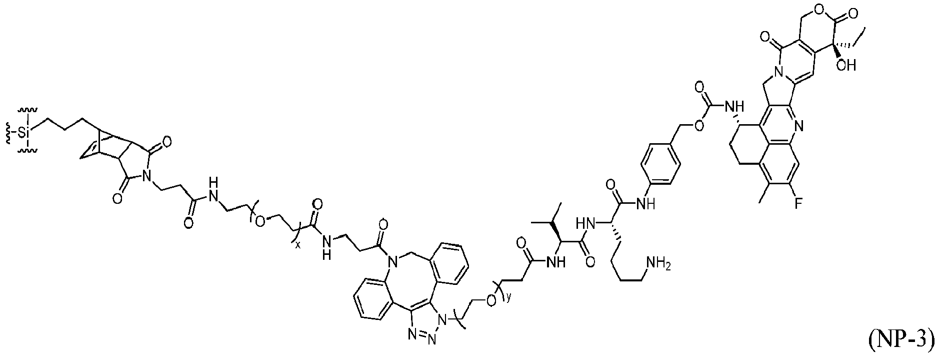 wherein x is an integer of 0 to 10 (e.g., 0, 1, 2, 3, 4, 5, 6, 7, 8, 9, or 10, e.g., 4), and y is an integer of 0 to 20 (e.g., 0, 1, 2, 3, 4, 5, 6, 7, 8, 9, 10, 11, 12, 13, 14, 15, 16, 17, 18, 19, or 20, e.g., 9), and the silicon atom is a part of the nanoparticle (e.g., bonded with the silica shell of a core-shell silica nanoparticle).

An exemplary NDC comprising a payload (exatecan) linked via a protease-cleavable linker, and the mechanism of cleavage of the linker and release of the payload, is depicted in Scheme 2.

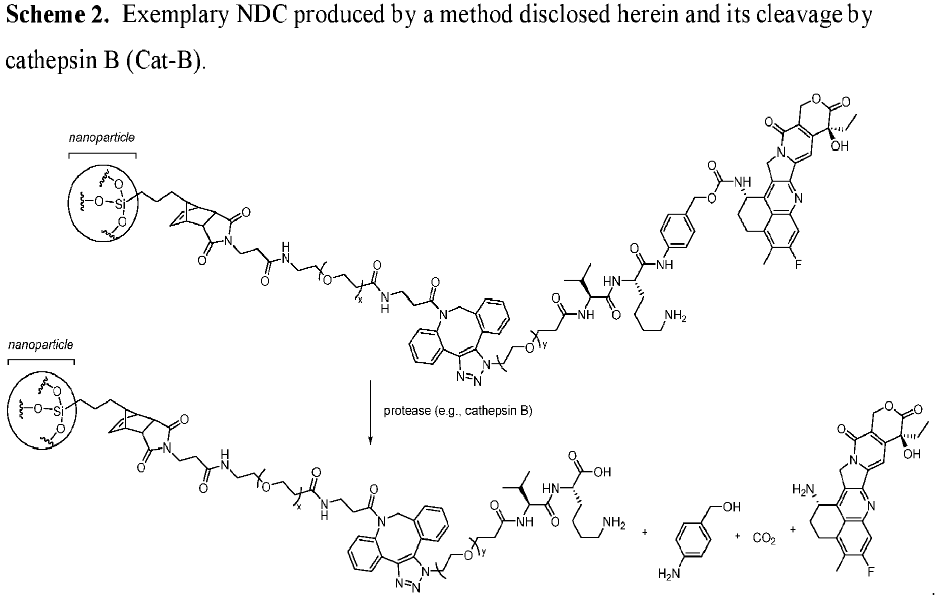

The methods disclosed herein can be used to obtain a nanoparticle drug conjugate with a desired number of targeting ligands or payload moieties. For example, an NDC produced using a method disclosed herein may have an average nanoparticle to payload (e.g., exatecan or a salt or analog thereof) ratio of about 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:32, 1:34, 1:36, 1:38, 1:40, 1:45, 1:50, 1:55, 1:60, 1:65, 1:70, 1:75, or 1:80. For example, the average number of exatecan molecules on each nanoparticle may be between about 5 and about 10, between about 10 and about 15, between about 15 and about 20, between about 20 and about 25, or between about 25 and about 30, e.g., about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 exatecan molecules per nanoparticle. An NDC produced using a method disclosed herein may have an average nanoparticle to targeting ligand (e.g., folic acid) ratio of about 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, or 1:30. For example, the average nanoparticle to targeting ligand ratio can range from about 1 to about 20, e.g., the average number of folic acid molecules on each nanoparticle may be between about 5 and about 10, between about 10 and about 15, or between about 15 and about 20, e.g., about 1, about 2, about 3, about 4, about 5, about 6, about 7, about 8, about 9, about 10, about 11, about 12, about 13, about 14, or about 15 folic acid molecules per nanoparticle.

It will be understood that while the methods exemplified herein feature functionalizing a nanoparticle with an alkyne, and conjugating a compound that comprises an alkyne-reactive moiety to the nanoparticle, these functionalities may be reversed. For example, the methods disclosed herein can be modified to functionalize the nanoparticle with an alkyne-reactive moiety (e.g., an azide, a diene, a nitrone, or a nitrile oxide), and further conjugate the functionalized nanoparticle with a compound comprising an alkyne. For example, the nanoparticle can be functionalized with a first bifunctional precursor, followed by conjugation to a second bifunctional precursor comprising an alkyne-reactive group, thereby providing a nanoparticle functionalized with an alkyne-reactive group (e.g., azide, a diene, a nitrone, or a nitrile oxide). Then, compounds (e.g., targeting ligands or payloads) comprising an alkyne may be reacted with the nanoparticle to conjugate the compounds to the nanoparticle.

The methods disclosed herein can overcome the shortcomings of other methods for functionalizing nanoparticles. For example, the methods disclosed herein can address the limited stability of nanoparticle conjugates made using other methods. In particular, the methods disclosed herein feature relatively stable bifunctional precursors, that do not promote the hydrolysis of bonds in the linker moieties, which can occur when using bifunctional precursors comprising highly reactive groups (e.g., certain amine groups). Additionally, the methods disclosed herein employ bifunctional precursors that do not substantially self-condense during reaction, which can occur when using other bifunctional precursors (e.g., amine-containing bifunctional precursors) due to, e.g., electrostatic attraction or other undesired intermolecular or intramolecular interactions. Overcoming these issues can greatly increase yield, and avoid difficult or impossible purification processes that would otherwise be required, which is particularly relevant for producing NDCs intended for clinical use. As such, the methods disclosed herein are particularly useful for producing NDCs intended for clinical use, due to their ease and reliability in producing highly pure functionalized nanoparticles.

Functionalized nanoparticles produced using methods disclosed herein can exhibit uniform morphology and narrow size distribution, e.g., as measured by transmission electron microscopy (TEM). The nanoparticles may be characterized by TEM, or UV spectroscopy, e.g., to identify absorption peaks characteristic of a dye or ligand in or on the nanoparticle. The purity of the nanoparticles can be assessed using chromatography, e.g., reversed-phase high performance liquid chromatography (RP-HPLC) and size-exclusion chromatography. The methods disclosed herein are also capable of providing highly consistent nanoparticle characteristics across various reaction scales, and batch-to-batch.

### EXAMPLES

In order that the invention described herein may be more fully understood, the following examples are set forth. These examples are offered to illustrate the methods and functionalized nanoparticles provided herein, and are not to be construed in any way as limiting their scope.

The compounds provided herein can be prepared from readily available starting materials using modifications to the specific synthesis protocols set forth below that would be well known to those of skill in the art. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvents used, but such conditions can be determined by those skilled in the art by routine optimization procedures.

Additionally, as will be apparent to those skilled in the art, conventional protecting groups may be necessary to prevent certain functional groups from undergoing undesired reactions. The choice of suitable protecting group for a particular functional group as well as suitable conditions for protection and deprotection are well known in the art. For example, numerous protecting groups, and their introduction and removal, are described in Greene et al. Protecting Groups in Organic Synthesis, Second Edition, Wiley, New York, 1991, and references cited therein.

### Materials and Methods

All materials were used as received. All non-aqueous reactions were run in flame-dried glassware under a positive pressure of argon. Anhydrous solvents were purchased from commercial suppliers (RANKEM). Flash chromatography was performed on 230-400 mesh silica gel with the indicated solvent systems. Proton Nuclear magnetic resonance spectra were recorded on Bruker Spectrometer at 400MHZ using deuterated chloroform or DMSO as solvent. Peak positions are given in parts per million downfield from tetramethylsilane as the internal standard. *J* values are expressed in hertz. Mass analyses were performed on (Agilent/Shimadzu) spectrometer using electrospray (ES) technique. HPLC analyses were performed on (Agilent/Waters), PDA-UV detector equipped with a Gemini C-18 (1000x 4.6 mm; 5u) and all compounds tested were determined to be >95% pure using this method. Compounds prepared according to the procedures described herein may be isolated by preparative HPLC methods. Cyanine5 maleimide was purchased from Lumiprobe. DBCO-PEG4-maleimide, folate-PEG-azide, and exatecan-linker conjugate were obtained from customer synthesis by WuXi AppTec, Tianjin, China. The (3-mercaptopropyl)trimethoxysilane, 2-[methoxy(polyethyleneoxy)6-9propyl]trimethoxysilane, and (3-cyclopentadienylpropyl)triethoxysilane precursors were purchased from Gelest. 10× PBS was purchased from Thermo Fisher Scientific. Human cathepsin-B was purchased from Millipore Sigma. Human and mouse serum were purchased from BIOIVT (New York). All other chemicals were purchased from Millipore Sigma. Cy5-C'Dot was prepared following the protocol described in Ma, K. et al. Chemistry of Materials (2015) 27(11):4119-4133, incorporated herein by reference in its entirety.

Gel permeation chromatography (GPC) characterization and purification was performed according to the procedures outlined in Chen, F. et al. Chemistry of Materials (2017) 29(20):8766-8779. Transmission electron microscopy (TEM) characterization was performed using a Titan Cubed Themis 300 TEM and high-angle annular dark-field (HAADF) imaging for demonstrating the distribution of nanoparticles. UV-Visible Spectroscopy analysis of samples was performed using a Cary 5000 UV-Vis-NIR spectrophotometer. Fluorescence Correlation Spectroscopy (FCS) measurements were performed using a home-built FCS setup as described in Ma, K. et al. Chemistry of Materials (2016) 28(5):1537-1545. A 633 nm solid-state laser was used to excite the Cy5 dye encapsulated in the silica core of C'Dots. RP-HPLC coupled to a photodiode array (PDA) detector was used to assess the purity of nanoparticle conjugates (including FA-CDC), using a commercially available Waters Xbridge Peptide BEH C18 column (50 × 4.6 mm). The mobile phase for the separation is a gradient elution starting at a composition of 85% A (0.1 vol % trifluoroacetic acid in deionized water)/15% B (acetonitrile) and the composition is linearly changed to 5%A/95%B over a course of 15 minutes. RP-HPLC separates molecules with different polarities and is suitable as an analytical method for FA-CDC because of its ultrasmall sub-10 nm particle size. Using RP-HPLC, the nanoparticles are well separated from other chemical moieties such as ligands that are non-covalently associated with the nanoparticles and degraded or force released components. Different chemical moieties are identified based on elution time and unique UV/Visible spectra collected using the PDA detector. The impurity content in a sample can be quantified using calibration curves developed with reference standard materials for key impurities. Size Exclusion Chromatography (SEC) characterization was performed using a Tosoh Bioscience PWXL 4000 7.8 mm x300 mm column coupled to a PDA detector, with a mobile phase composed of 80% 0.9 wt. % sodium chloride in deionized water and 20% acetonitrile. The separation occurs over the course of 30 minutes in the isocractic mode, and the typical flow rate for the separation is 0.6 mL/min.

### Example 1. Synthesis of an exemplary functionalized nanoparticle using DBCO-PEG-silane.

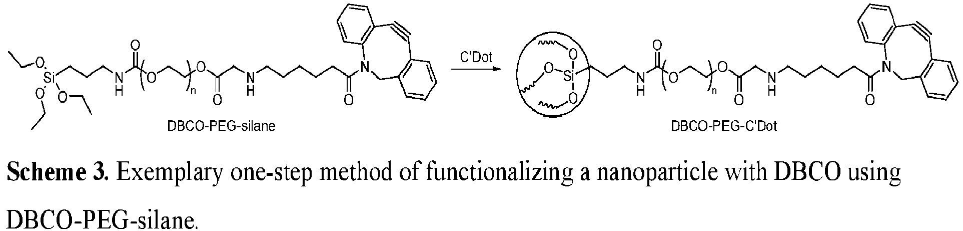

Cy5-C'Dot was diluted with deionized water to a desired concentration, typically between 15 to 30 µM, in a round-bottom flask with a stir bar. DBCO-PEGn-silane (n=4 or 12) was dissolved in DMSO and added into the reaction under stirring, to achieve a desired particle to DBCO molar ratio. After reaction at room temperature overnight, the reaction solution was concentrated and purified using gel permeation chromatography (GPC). The number of DBCO groups attached to the nanoparticles is provided in FIG. 2.

### Example 2. Synthesis of an exemplary functionalized nanoparticle using amine-based precursor.

Silanol groups on the surface of the nanoparticle under the organic polymer coating (e.g., PEG layer) are not easily accessible to bulky molecules, such as DBCO-PEG-silane. The result is that when using DBCO-PEG-silane as a bifunctional precursor (e.g., as in Example 1), only one or two DBCO groups can be attached to each nanoparticle (e.g., C'Dot), even at a high reaction ratio of DBCO-PEG-silane to nanoparticle (see FIG. 2). Therefore, an alternative two-step reaction was designed, employing relatively small silane molecules with a high diffusion coefficient, depicted in Scheme 4.

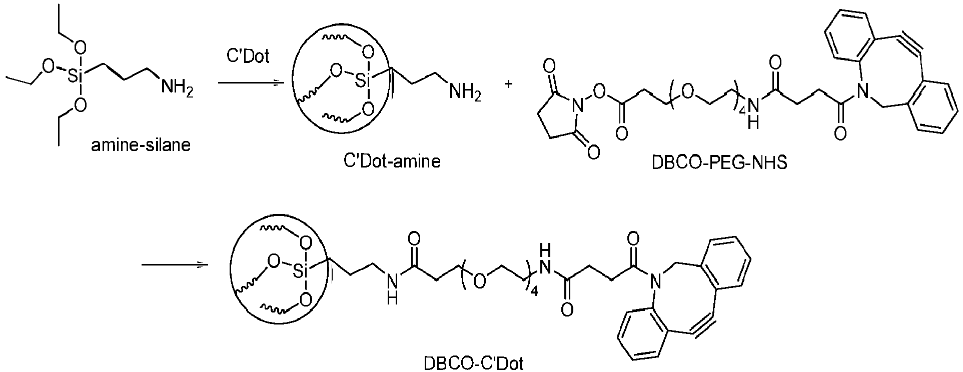

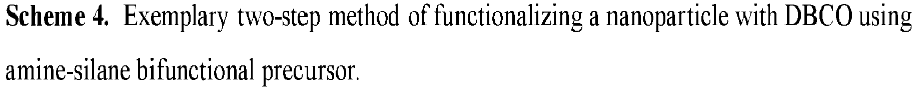

Cy5-C'Dot was diluted with deionized water to a desired concentration, typically between 15 to 30 µM, in a round-bottom flask with a stir bar. (3-Aminopropyl)triethoxysilane (APTMS) was added into the C'Dot solution under stirring, to achieve a desired particle to APTMS molar ratio. After overnight reaction, DBCO-PEG4-NHS ester in DMSO was added to reach a desired particle to DBCO molar ratio, and to further conjugate to the amine groups on C'Dots via amine-ester reaction. The reaction was left at room temperature overnight, and then concentrated and purified using GPC. After GPC purification, the purified particles in 0.9% saline were heated to 80 °C in water bath overnight. After heat treatment, the solution was concentrated and purified again using GPC to obtain amine-based DBCO-C'Dots. In comparison to the single-step reaction, this two-step reaction makes use of the fast diffusion of small silane molecules to enhance their reactivity to the silanol groups under the PEG layer, permitting more than 20 DBCO groups to be easily attached onto each C'Dot (see FIG. 2).

However, the amine-based DBCO-C'Dots produced using this approach exhibited limited stability. For example, a considerable amount of DBCO detached from C'Dots after incubation in PBS at 37 °C overnight (see FIG. 3A), which may be due to the hydrolysis of the amide bonds between the DBCO groups and C'Dot, accelerated by residual primary amine groups on the C'Dot surface. Additionally, amine-silane molecules can self-condense during reaction due to the electrostatic attraction between their positively charged amine portion and negatively charged silanol portion. Amine-silane aggregates are difficult to remove from C'Dot solution by size-exclusion chromatography (SEC) (see FIG. 3C), and can lead to undesired downstream effects. For example, if administered, these aggregates may lead to kidney retention due to the positive charge of amine.

### Example 3. Screening Conjugation Chemistry Synthesis

To overcome the shortcomings of using amine-silane precursors described in Example 2, a library of conjugation chemistry was screened, which is summarized in Table 1.

**Table 1. Screening of conjugation chemistry.**

| **Conjugation Chemistry** | | **No. of ligands per particle** |
|---|---|---|
| **1^{st} Step** | **2^{nd} Step** | |
| Amine-silane | Azide-PEG4-NHS | Up to 4^{a} |
| | Alkyne-PEG4-NHS | Not accessible^{b} |
| | DBCO-PEG4-NHS | >40^{c} |
| Diene-silane | DBCO-maleimide | >40^{c} |
| | DBCO-sulfo-maleimide | Upto 10 |
| | DBCO-PEG4-maleimide | >40^{c} |
| | DBCO-PEG12-maleimide | >40^{c} |
| Azide-silane | | Up to 1.4^{d} |
| DBCO-PEG4-silane | | Up to 2 |
| DBCO-PEG12-silane | | Up to 2 |

| | | |
|---|---|---|
| ^{a}The number of azide groups per particle cannot be directly quantified from UV-Vis spectra. Therefore, DBCO-Cy3 molecules were further conjugated to azide-functionalized C'Dots, attaching Cy3 to particles via click chemistry reaction between DBCO and azide; ^{b} The presence of alkyne groups on C' dot was verified by the specific absorbance peak of alkyne at 290 nm; ^{c}Obtained after optimization of synthesis protocol; ^{d} DBCO-Cy3 molecules were further conjugated to azide-functionalized C'Dots, attaching Cy3 to particles via click chemistry reaction between DBCO and azide. | | |

### Example 4. Synthesis of a diene-based DBCO-C'Dot

The use of a Diels-Alder reaction was identified in Example 3 as a suitable replacement for the amine-ester reaction. For example, the amine-silane used in the method described in Example 2 can be replaced with (3-cyclopentadienylpropyl)triethoxysilane ("diene-silane") to first functionalize C'Dots with cyclopentadiene groups, and DBCO-PEG-NHS ester used in the method of Example 2 can be replaced with a DBCO-PEG-maleimide. This method is depicted in Scheme 5, where a Diels-Alder reaction between the maleimide groups on DBCO-PEG-maleimide and the diene groups on C'Dots can be used to attach the DBCO (via a linker) to the nanoparticle.

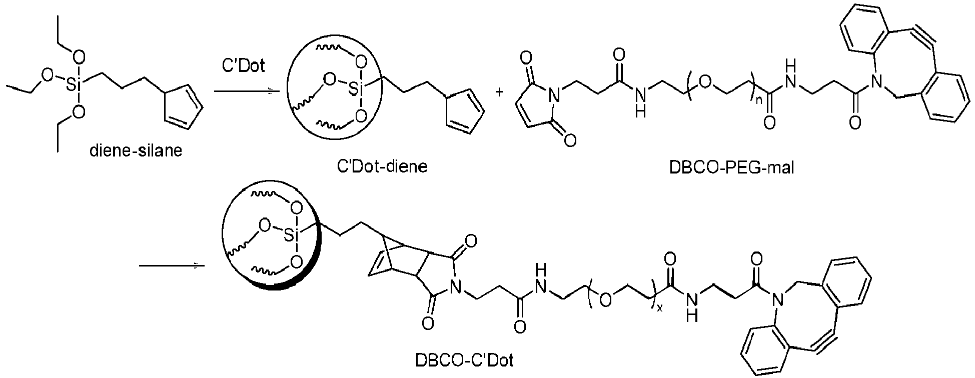

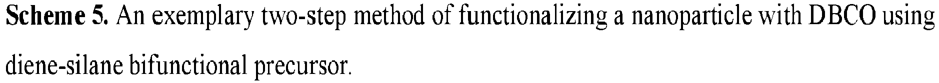

Cy5-C'Dot was diluted with deionized water to a desired concentration, typically between 15 to 30 µM, in a round-bottom flask with a stir bar. (3-Cyclopentadienylpropyl)triethoxysilane (cyclopentadiene) was first diluted 100x in DMSO and then added into the reaction under stirring, to reach a desired particle to cyclopentadiene molar ratio. After overnight reaction, 10× PBS was added into the reaction to achieve a final concentration of 1× PBS. Next, a DBCO-maleimide precursor (DBCO-mal, DBCO-PEG4-mal, DBCO-PEG12-mal, or DBCO-sulfo-mal) was dissolved in DMSO and added into the reaction to reach a desired particle to DBCO molar ratio. After mixing for about 30 min to 1 hour, the reaction mixture was heated to 80 °C while stirring overnight. The reaction solution was then concentrated and purified using gel permeation chromatography (GPC) to obtain diene-based DBCO-C'Dot.

Without wishing to be bound by theory, it is believed that the neutral charge of the cyclopentadiene groups averts hydrolysis of the amide bonds in the linkage, that can be accelerated by the primary amine on C'Dots when using an amine-silane as a precursor. Thus, the C'Dots produced using this method are highly stable (see FIG. 3B). Additionally, using cyclopentadiene groups greatly diminishes the self-condensation of silane during the reaction (see FIG. 3D), and improves the stability, size homogeneity, reaction yield, and purity of the functionalized nanoparticles (e.g., DBCO-C'Dots).

The reaction efficiency of the DBCO-containing precursor (e.g., DBCO-maleimide) is independent of its diffusion coefficient. For example, even when DBCO-maleimide precursors with varying PEG spacer lengths and molecular weights were employed in functionalizing C'Dots, the number of DBCO groups attached to each of the resulting C'Dots remained consistent (see FIG. 2). This is a departure from typical methods involving other bifunctional precursors, where the reaction efficiency can be greatly influenced by the molar mass of silanes. This also suggests that low yields from reacting C'Dots with bulky silanes is mainly caused by the self-condensation of silanes, which competes with their diffusion into the organic polymer coating of the nanoparticle (e.g., the C'Dot PEG layer) and with subsequent reactions. Reaction between DBCO-maleimide precursor and nanoparticle (e.g., C'Dot) can be sensitive to the hydrophobicity and charge status of the DBCO-maleimide precursor used. For example, relatively hydrophilic molecules with negative charges, e.g. DBCO-sulfo-maleimide with a hydrophilic sulfonated spacer, exhibits relatively poor reactivity. Without wishing to be bound by theory, this poor reactivity may be due to the increased water solubility and electrostatic repulsion from the negatively charged silanol groups. Based on these results, a short PEG spacer with 4 ethylene glycol units was used in a DBCO-maleimide precursor to endow the DBCO groups on C'Dot surface with the desired accessibility for subsequent modification, e.g., using click chemistry reactions.

### Example 5. Synthesis of FA-CDC

NDCs comprising an alkyne moiety (e.g., DBCO) produced by the methods disclosed herein can be further conjugated with a compound comprising a reactive group, e.g., an alkyne-reactive group (e.g., an azide). These compounds can include payload-linker conjugate precursors, e.g., as described in Synthetic Example 1, and/or folate targeting ligand precursors, e.g., as described in Synthetic Example 2.

For example, using click chemistry, the DBCO-C'Dots produced by the methods disclosed herein were conjugated with azide-functionalized folic acid-linker conjugates (suitable as a targeting ligand for targeting cancers expressing folate receptor (FR)), and azide-functionalized exatecan-linker conjugates (exatecan is a topoisomerase I inhibitor drug, suitable for treating cancer). The folic acid moiety was attached through a non-cleavable linker, while the exatecan moiety was attached through a cathepsin-B (Cat-B) cleavable linker (Cat-B is an enzyme present in cell lysosomes and overexpressed in a variety of malignancies). These methods are depicted in Scheme 6.

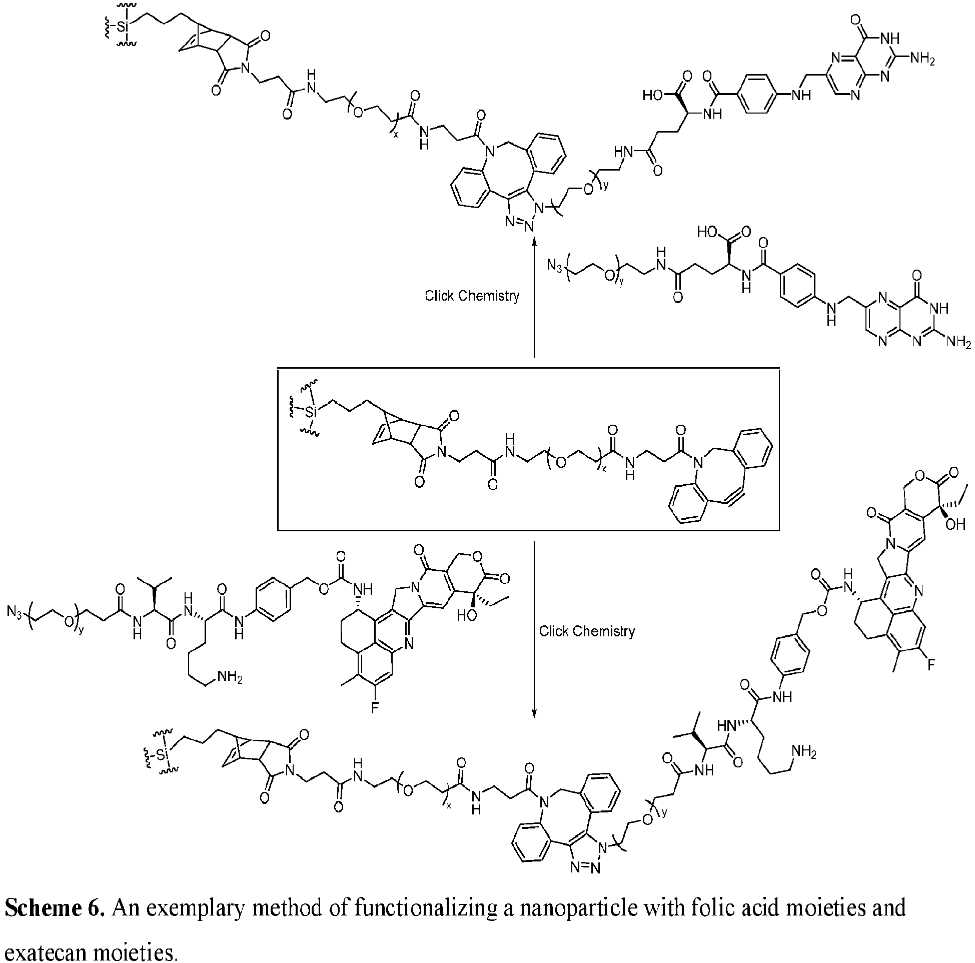

DBCO-C'Dot was diluted with deionized water to a desired concentration, typically between 15 to 30 µM, in a round-bottom flask with a stir bar. To functionalize DBCO-C'Dot with folic acid ligands, folate-PEG-azide was dissolved in DMSO and added into the reaction under stirring, to achieve a desired number of ligands per particle. The reaction was left at room temperature overnight, and the conversion rate was checked by in-process HPLC purity test (typically above 95%). If the purity was below 95%, the reaction solution would be concentrated and purified using GPC. The folic acid functionalized C'Dot was isolated as an intermediate to determine the number of folic acid ligands per particle using UV-Vis. To further conjugate folic acid functionalized C'Dot with cleavable exatecan, the particle concentration was adjusted as needed. Cleavable linker-exatecan conjugate was dissolved in DMSO and added into the particle solution under stirring, to achieve a desired number of payloads per particle. The reaction was left at room temperature overnight, and then reaction solution was concentrated and purified using GPC to obtain FA-CDC.

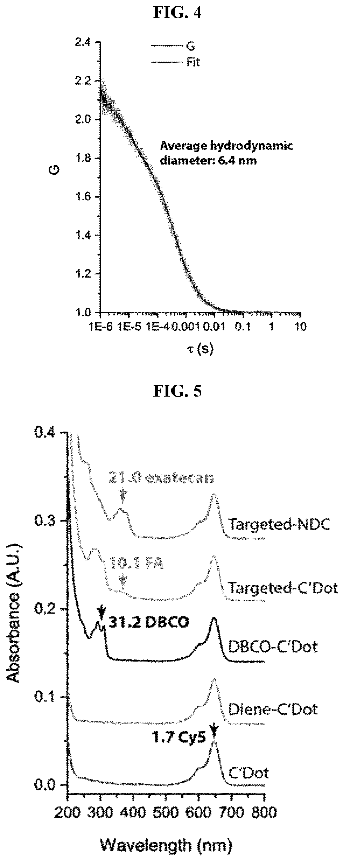

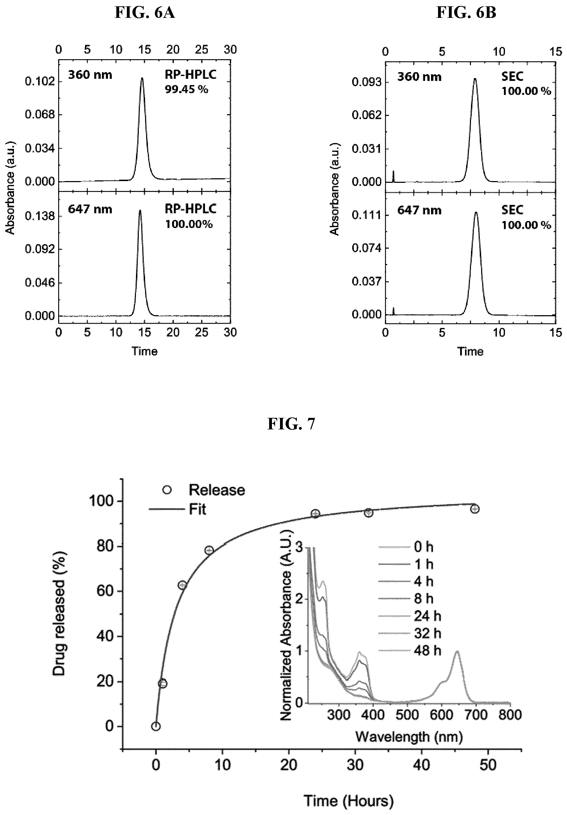

The resulting targeted-NDCs exhibited uniform morphology and narrow size distribution under transmission electron microscopy (TEM) with an average core size of 3.9 nm. The average hydrodynamic diameter of the NDCs was determined by fluorescence correlation spectroscopy (FCS) to be 6.4 nm (FIG. 4), consistent with TEM as the 2.5 nm difference is attributed to the organic PEG layer that cannot be observed in TEM. The final purified NDCs had a purity above 99.0 % as characterized by both reversed-phase high performance liquid chromatography (RP-HPLC) and SEC (FIGS. 6A-6B). These quality attributes of NDCs were demonstrated to be highly consistent from batch to batch across various reaction scales (Table 2).

**Table 2. Characteristics of NDCs from batch to batch at exemplary scales.**

| **Specifications** | | **Test methods** | **Batch sizes and Lot Information** | | | |
|---|---|---|---|---|---|---|
| | | | **30 mL** | **75 mL** | **1 L** | **12 L** |
| Appearance | | Visual inspection | Clear blue | Clear blue | Clear blue | Clear blue |
| Identity - Absorbance | | UV-Vis | Conforms | Conforms | Conforms | Conforms |
| Identity - HPLC elution time | | RP-HPLC | Conforms | Conforms | Conforms | Conforms |
| Average particle size | | FCS | 6.0 nm | 5.8 nm | 6.2 nm | 6.6 nm |
| Number of FR-targeting ligands per particle | | Enzymatic digestion followed by RP-HPLC | 15.2 | 13.8 | 12.3 | 13.3 |
| Number of cleavable exatecan per particle | | UV-Vis | 19.9 | 23.3 | 21.5 | 17.7 |
| Purity | | RP-HPLC | 100.00 % | 99.82 % | 99.28 % | 99.33 % |

| Impurities | | | | | | |
|---|---|---|---|---|---|---|
| | Exatecan | RP-HPLC | < 0.02 % | 0.18 % | < 0.02 % | < 0.02 % |
| | Cleavable exatecan | | < 0.05 % | < 0.02 % | 0.72 % | 0.67 % |
| | Folic acid | | < 0.05 % | 0.12 % | < 0.05 % | < 0.05 % |
| Aggregation | | SEC | < 0.05 % | 0.06 % | < 0.05 % | < 0.05 % |
| Drug Release Profile | | Enzymatic digestion followed by RP-HPLC | 71.8 % | 69.6 % | 81.9 % | 81.7 % |

UV-Vis spectra were collected at each step when C'Dots were sequentially functionalized with cyclopentadiene, DBCO, folic acid, and exatecan to produce targeted-NDC. The characteristic absorption peaks were used to verify the presence of each ligand and to determine the number of ligands per particle (FIG. 5). DBCO-C'Dot exhibits one absorption peak at 647 nm corresponding to covalently encapsulated Cy5, and a doublet peak at 308 nm and 290 nm corresponding to DBCO groups. The molar concentration of Cy5 and DBCO can be determined by application of the Beer-Lambert Law at 647 nm and 308 nm respectively using their extinction coefficients. Particle concentration was obtained by dividing the molar concentration of Cy5 by the number of Cy5 dyes per particle as determined by FCS. The number of DBCO groups per particle was obtained by dividing the molar concentration of DBCO groups by particle concentration.

The UV-Vis spectrum of folic acid functionalized C'Dot before attaching exatecan payloads was used to determine the concentration of the particles and the concentration of FR-targeting ligands by application of the Beer-Lambert Law at 647 nm (corresponding to covalently encapsulated Cy5) and 360 nm (corresponding to folic acid targeting ligands), respectively. The number of FR-targeting ligands per particle was determined by dividing the molar concentration of FR-targeting ligands by the particle concentration. The number of FR-targeting ligands have been confirmed to remain unchanged in subsequent processing to produce FA-CDC.

Similarly, the UV-Vis spectrum of FA-CDC can be used to determine the concentration of FA-CDC particles, as well as the concentration of cleavable exatecan by application of the Beer-Lambert Law at 647 nm and 360 nm (corresponding to both folic targeting ligands and exatecan) after subtraction of the absorbance of FR-targeting ligands at 360 nm. The concentration of exatecan was then divided by the concentration of FA-CDC particles to determine the number of exatecan per particle.

### Example 6. Stability of NDCs in saline, PBS, human, and mouse serum

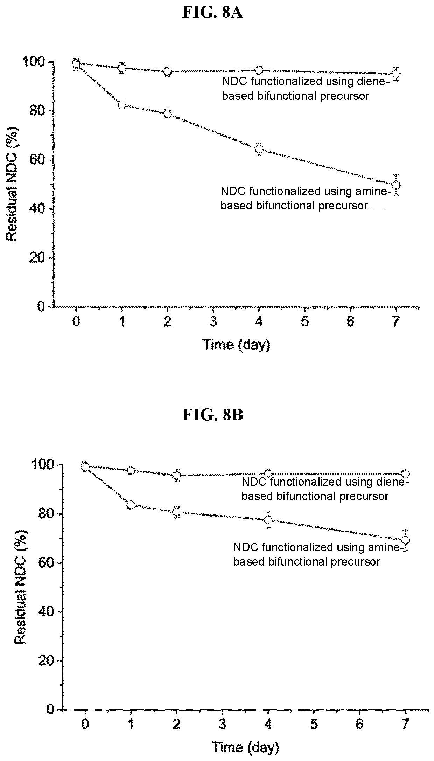

The stability of NDCs produced herein was assessed after incubation in 0.9% saline, PBS, human plasma (10%), and mouse plasma (10%) at 37 °C in a shaking dry bath for different time periods. Prior to analysis, plasma proteins in the samples were removed by precipitation, through addition of an equivalent volume of cold acetonitrile, followed by centrifugation at 10000 rpm in an Eppendorf 5425 microcentrifuge. Following centrifugation, the clear supernatant was transferred from the centrifuge tube into a clear total recovery HPLC vial. The supernatant free of any visible aggregation was diluted with an equivalent volume of deionized water to adjust the sample matrix to match the starting conditions of the HPLC separation and avoid loss of sensitivity. The purity and impurity of each sample is then quantified by RP-HPLC as described above. Stability in human and mouse serum of NDCs produced by a method of Example 2 and Example 4 are provided in FIGS. 8A-8B.

Nanoparticle conjugate samples (e.g., FA-CDC) were prepared for forced release by first diluting an aliquot of the FA-CDC sample to 2 µM concentration and then incubating with activated recombinant human Cathepsin-B in a shaking dry bath at 37° C. Recombinant human Cathepsin-B was prepared as follows: 4 µL of 0.33 µg/µL Cathepsin-B was added to an activation buffer consisting of dithiothreitol and (MES) and adjusted to pH 5.0. Appropriate pH was confirmed with a pH test strip after preparation. After 24 hours of incubation, the force released free exatecan is determined by the percentage of the integrated area that is present at the free exatecan elution time in RP-HPLC. The results of this study are provided in FIG. 7.

When the targeted-NDCs were incubated with recombinant human Cat-B (SI), more than 95% of exatecan drugs were released from the NDCs within 48 hours with a half-life around 3.0 hours from fitting (FIG. 7), demonstrating the effective drug release in cancer environment. The targeted-NDCs, produced using the diene-silane precursor (depicted in Scheme 5) also exhibited high stability in mouse and human plasma and showed significant stability improvement (FIGS. 8A and 8B), relative to their counterparts produced using an amine-silane precursor (depicted in Scheme 4). More than 95% of exatecan drugs remain on the NDCs for up to 7 days in mouse and human plasma, obtained by the UV-Vis spectra of the NDC peaks in RP-HPLC chromatograms. Meanwhile, an independent RP-HPLC assay monitoring free exatecan suggested that the released exatecan was below detection limit of RP-HPLC, i.e., 0.02%, and the absence of non-desired free drug further demonstrates their high plasma stability. The targeted-NDCs also exhibited high storage stability at 4 °C in 0.9% saline. Their purity, size distribution, and hydrodynamic diameter were characterized by RP-HPLC, SEC and FCS respectively, and remained unchanged over 6 months under storage condition. Such high storage stability is another key parameter important for both clinical translation and commercial manufacture.

### Synthetic Example 1: Synthesis of Linker-Payload Conjugate Precursors

Linker-payload conjugate precursors suitable for use in the methods disclosed herein can be synthesized according to the following exemplary protocols.

### Synthesis of (S)-2-amino-N-(4-(((tert-bulyldiphenylsilyl)oxy)methyl)phenyl)-6-((diphenyl(p tolyl)methyl)amino)hexanamide (161)

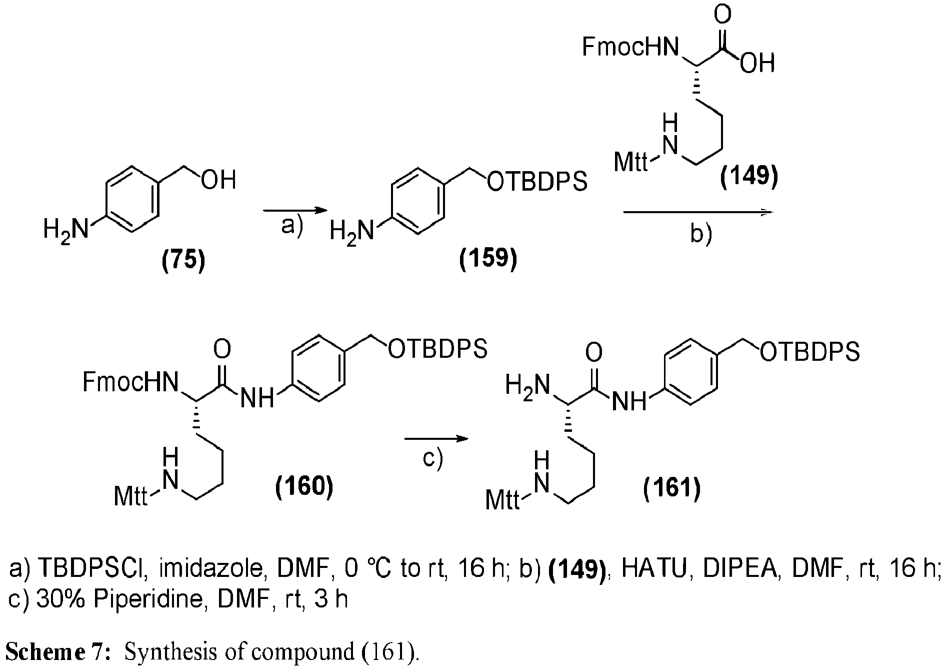

*Synthesis of 4-(((tert-butyldiphenylsilyl)oxy)methyl)aniline (159):* Imidazole (5.54 g, 81.22 mmol) was added to a solution of (4-aminophenyl)methanol (75) (5.0 g, 40.61 mmol) in DMF (25 mL) at 0°C, followed by *tert*-butyl(chloro)diphenylsilane (13.39 g, 48.73 mmol), and the reaction mixture was stirred at room temperature for 16 h. The progress of the reaction was monitored by TLC. After completion of starting material, the reaction mixture was quenched with water (20 mL) and extracted with EtOAc (2x200 mL). The combined organic layers were dried over anhydrous Na₂SO₄, concentrated under reduced pressure, and purified by column chromatography using silica gel (230-400 mesh) eluting with 10% EtOAc in petroleum ether to afford 4-(((*tert*-butyldiphenylsilyl)oxy)methyl)aniline (159; 6.6 g) as a gum. LCMS: *m*/*z* 362.31 [(M+H)⁺]; *Rₜ*: 2.58 min; 93.68% purity.

*Synthesis of (9H-fluoren-9-yl)methyl(S)-(1-((4-(((tert-butyldiphenylsilyl)oxy)methyl)phenyl)amino)-6-((diphenyl(p-tolyl)methyl)amino)-1-oxohexan-2-yl)carbamate (160):* Diisopropylethylamine (4.18 mL, 24 mmol), HATU (6.08 g, 16 mmol) and 4-(((tert-butyldiphenylsilyl)oxy)methyl)aniline (159) (2.89 g, 8 mmol) were added to a solution of N2-(((9H-fluoren-9-yl)methoxy)carbonyl)-N6-(diphenyl(*p*-tolyl)methyl)-L-lysine (149) (5.0 g, 8 mmol) in DMF (50 mL)at 0 °C, and the reaction mixture was stirred at room temperature for 16 h. The progress of the reaction was monitored by TLC. After completion of starting material, the reaction mixture was quenched with ice water. The precipitated solid was filtered and dried under vacuum to afford (9H-fluoren-9-yl)methyl (*S*)-(1-((4-(((*tert-*butyldiphenylsilyl)oxy)methyl)phenyl)amino)-6-((diphenyl(p-tolyl)methyl)amino)-1-oxohexan-2-yl)carbamate (160; 5.5 g) as a solid. LCMS: *m*/*z* 990.37 [(M+H)⁺]; *Rₜ*: 2.84 min; 96.79% purity.

*Synthesis of (S)-2-aminio-N-(4-(((tert-butyldiphenylsilyl)oxy)methyl)phenyl)-6-((diphenyl(p tolyl)methyl)amino)hexanamide (161):* Piperidine (16.5 mL) was added to a solution of (9H-fluoren-9-yl)methyl (*S*)-(1-((4-(((*tert-*butyldiphenylsilyl)oxy)methyl)phenyl)amino)-6-((diphenyl(p-tolyl)methyl)amino)-1-oxohexan-2-yl)carbamate (160) (5.5 g, 5.68 mmol) in DMF (38.5 mL) at room temperature, and the reaction mixture was stirred at room temperature for 3 h. The progress of the reaction was monitored by TLC. After completion of starting material, the reaction mixture was concentrated under reduced pressure, and purified by column chromatography using silica gel (230-400 mesh) eluting with 100% EtOAc to afford (*S*)-2-amino-N-(4-(((*tert-*butyldiphenylsilyl)oxy)methyl)phenyl)-6-((diphenyl(*p*-tolyl)methyl)amino)hexanamide (**160**; 3.5 g) as a gum. LCMS: *m*/*z* 744.24 [(M-H)⁻]; *Rₜ*: 2.20 min; 90.16% purity.

### Synthesis of 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl(4-nitrophenyl) carbonate (191).

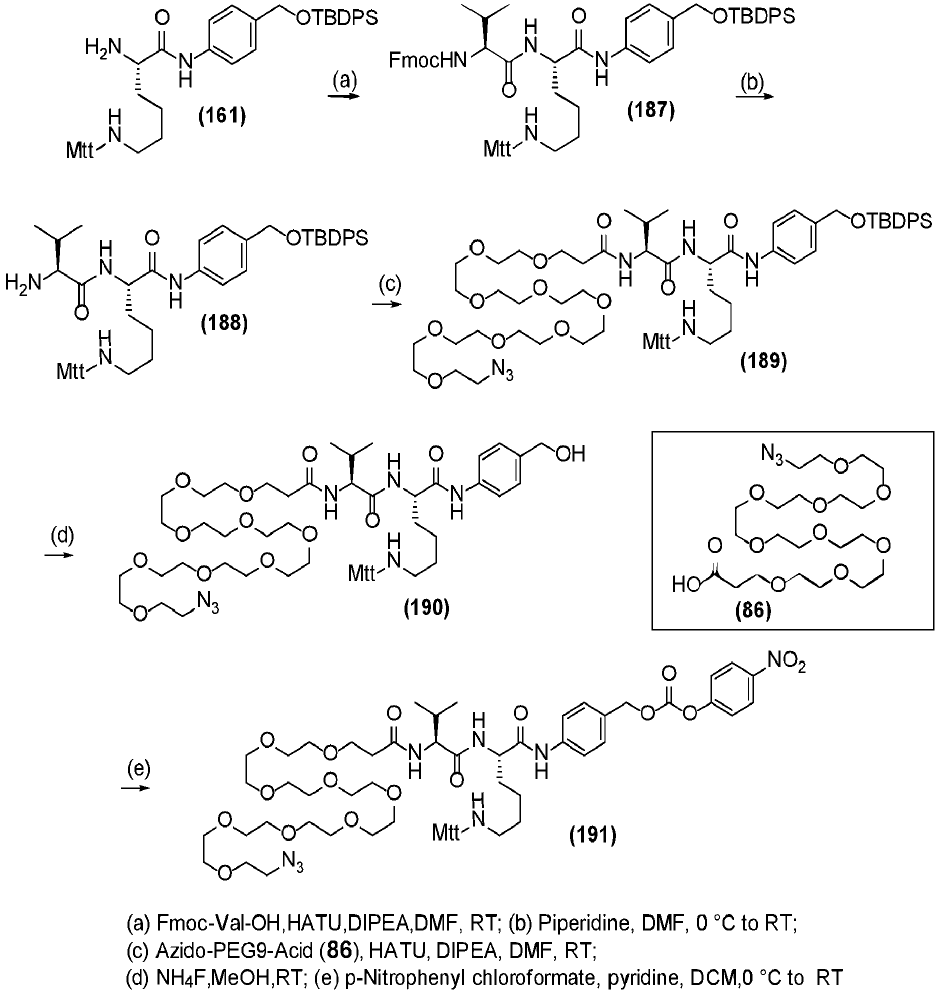

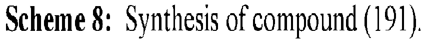

*Synthesis of (9H Fluoren-9-yl)methyl((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)carbamate (187)*: Diisopropylethylamine (1.54 mL, 8.83 mmol), HATU (2.24 g, 5.89 mmol) and (S)-2-amino-N-(4-(((tert-butyldiphenylsilyl)oxy)methyl)phenyl)-6-((diphenyl(p-tolyl)methyl)amino)hexanamide (161) (2.19 g, 2.94 mmol) were added to a solution of (((*N*-(9-Fluorenylmethoxycarbonyl)-L-valine (1 g, 2.94 mmol), in DMF (20 mL)at 0°C, and the reaction mixture was stirred at room temperature for 3 h. The progress of the reaction was monitored by TLC. After completion of starting material, the reaction mixture was quenched with ice water. The precipitated solid was filtered and dried under vacuum to afford (((9H-fluoren-9-yl)methyl ((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)carbamate (187; 2.5 g) as a solid. LCMS: MH⁺ 1067, retention time 2.42 min.

*Synthesis of (S)-2-((S)-2-Amino-3-methylbutanamido)-N-(4-(((tert-butyldiphenylsilyl)oxy)methyl)phenyl)-6-((diphenyl(p-tolyl)methyl)amino)hexanamide (188):* A 30% solution of piperidine in DMF (4.5 mL) was added to a solution of (((9H-fluoren-9-yl)methyl ((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)carbamate **(187)** (1.5 g, 1.40 mmol) in DMF (6 mL) at room temperature, and the reaction mixture was stirred at room temperature for 2 h. The progress of the reaction was monitored by TLC. After completion of starting material, the reaction mixture was concentrated under reduced and purified by flash chromatography eluting with 100% EtOAc, to afford (S)-2-((S)-2-amino-3-methylbutanamido)-N-(4-(((tert-butyldiphenylsilyl)oxy)methyl)phenyl)-6-((diphenyl(p-tolyl)methyl)amino)hexanamide (188; 1.1 g) as a solid. ¹H NMR (400 MHz, DMSO-*d*₆): *δ* 10.07 (s, 1H), 7.64-7.63 (d, 4H), 7.56-7.54 (d, 2H), 7.46-7.35 (m, 9H), 7.27-7.24 (m, 8H), 7.185-7.11 (m, 2H), 7.05-7.03 (d, 2H), 4.71 (s, 2H), 4.44 (d, 1H), 3.25-3.16 (d, 1H), 3.01-3.00 (m, 1H), 2.21 (s, 3H), 1.98-1.93 (m, 2H), 1.68-1.38 (m, 4H), 1.15 (s, 10H), LCMS: MH⁺ 845, retention time 3.63 min.

*Synthesis of 1-azido-N-((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)-3,6,9,12,15,18,21,24,27-nonaoxatriacontan-30-amide* (***189***): Diisopropylethylamine (0.49 mL, 2.83 mmol), HATU (719.47 mg, 1.89 mmol) and (S)-2-((S)-2-amino-3-methylbutanamido)-N-(4-(((tert-butyldiphenylsilyl)oxy)methyl)phenyl)-6-((diphenyl(p-tolyl)methyl)amino)hexanamide (188) (800 mg, 0.94 mmol) were added to a solution of 1-azido-3,6,9,12,15,18,21,24,27-nonaoxatriacontan-30-oic acid (86) (484 mg, 0.94 mmol) in DMF (8 mL)at 0 °C, and the reaction mixture was stirred at room temperature for 6 h. The progress of the reaction was monitored by TLC. After completion of starting material, the reaction mixture was quenched with water (15 mL) and extracted with EtOAc (2x30 mL). The combined organic layers were dried over anhydrous Na₂SO₄, concentrated under reduced, and purified by flash chromatography eluting with 3% MeOH in DCM to provide 1-azido-N-((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)-3,6,9,12,15,18,21,24,27-nonaoxatriacontan-30-amide (189; 0.60 g) as a gum. ¹H NMR (400 MHz, DMSO-*d*₆): *δ* 9.91 (s, 1H), 8.02-8.00 (d, 2H), 7.95 (s, 2H), 7.87-7.85 (d, 1H), 7.64-7.63 (d, 4H), 7.57-7.55 (d, 2H), 7.46-7.32 (m, 11H), 7.26-7.24 (m, 8H), 7.15-7.11 (t, 2H), 7.05-7.03 (d, 2H), 4.71 (s, 2H), 4.35-4.33 (m, 1H), 4.19 (s, 1H), 3.59-3.36 (m, 38H), 2.68-2.38 (m, 6H), 2.22 (s, 3H), 1.98-1.92 (m, 2H), 1.47-1.17 (m, 4H), 1.02 (s,9H), 0.85-0.80 (m, 6H). LCMS: MH⁺ 1338, retention time 2.92 min.

*Synthesis of 1-azido-N-((S)-1-(((S)-6-((diphenyl(p-tolyl)methyl)amino)-1-((4-(hydroxymethyl)phenyl)amino)-1-oxohexan-2-yl)amino)-3-methyl-1-oxobutan-2-yl)-3,6,9,12,15,18,21,24,27-nonaoxatriacontan-30-amide (190):* NH₄F (166 mg, 4.48 mmol) was added to a solution of 1-azido-N-((S)-1-(((S)-1-((4-(((tert-butyldiphenylsilyl)oxy)methyl)phenyl)amino)-6-((diphenyl(p-tolyl)methyl)amino)-1-oxohexan-2-yl)amino)-3-methyl-1-oxobutan-2-yl)-3,6,9,12,15,18,21,24,27-nonaoxatriacontan-30-amide (189) (600 mg, 0.44 mmol) in methanol (10 mL) at room temperature, and the reaction mixture was stirred at room temperature for 6 h. The progress of the reaction was monitored by TLC. After completion of starting material, the reaction mixture was concentrated under reduced pressure, and the residue obtained was diluted with water (15 mL) and extracted with EtOAc (2x20 mL). The combined organic layers were dried over anhydrous Na₂SO₄, concentrated under reduced pressure, and purified by flash chromatography eluting with 5 % MeOH in DCM to afford 1-azido-N-((S)-1-(((S)-6-((diphenyl(p-tolyl)methyl)amino)-1-((4-(hydroxymethyl)phenyl)amino)-1-oxohexan-2-yl)amino)-3-methyl-1-oxobutan-2-yl)-3,6,9,12,15,18,21,24,27-nonaoxatriacontan-30-amide (190; 0.40 g) as a gum. ¹H NMR (400 MHz, DMSO-*d*₆): *δ* 9.81 (s, 1H), 7.96-7.94 (d, 1H), 7.84-7.81 (d, 1H), 7.53-7.51 (d, 2H), 7.37-7.35 (d, 4H), 7.26-7.12 (m, 9H), 7.096-7.04 (d, 2H), 5.06-5.04 (t, 1H), 4.43-4.41 (d, 2H), 4.35 (m, 1H), 4.18-4.16 (t, 1H), 3.60-3.46 (m, 33H), 3.39-3.36 (t, 2H), 2.50-2.23 (m, 2H), 2.23 (s, 3H), 2.23-1.93 (m,2H), 1.48-1.23 (m, 6H), 0.85-0.80 (m, 6H). LCMS: MH⁺ 1100, retention time 3.72 min.

*Synthesis of 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl(4-nitrophenyl)carbonate (191):* Pyridine (0.14 mL, 1.80 mmol) and 4-nitrophenyl chloroformate (14) (145 mg, 0.72 mmol) were added to a solution of 1-azido-N-((S)-1-(((S)-6-((diphenyl(p-tolyl)methyl)amino)-1-((4-(hydroxymethyl)phenyl)amino)-1-oxohexan-2-yl)amino)-3-methyl-1-oxobutan-2-yl)-3,6,9,12,15,18,21,24,27-nonaoxatriacontan-30-amide (190) (400 mg, 0.36 mmol) in DCM (10 mL)at 0 °C, and the reaction mixture was stirred at room temperature for 6 h. The progress of the reaction was monitored by TLC. After completion of starting material, the reaction mixture was concentrated under reduced pressure, and purified by flash chromatography eluting with 3% MeOH in DCM to afford 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl (4-nitrophenyl) carbonate (191; 0.34 g) as a gum. LCMS: MH⁺ 1265, retention time 1.33 min.

### Synthesis of 4-((32S,35S)-35-(4-aminobutyl)-1-azido-32-isopropyl-30,33-dioxo-3,6,9,12,15,18,21,24,27-nonaoxa-31,34-diazahexatriacontan-36-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (202).

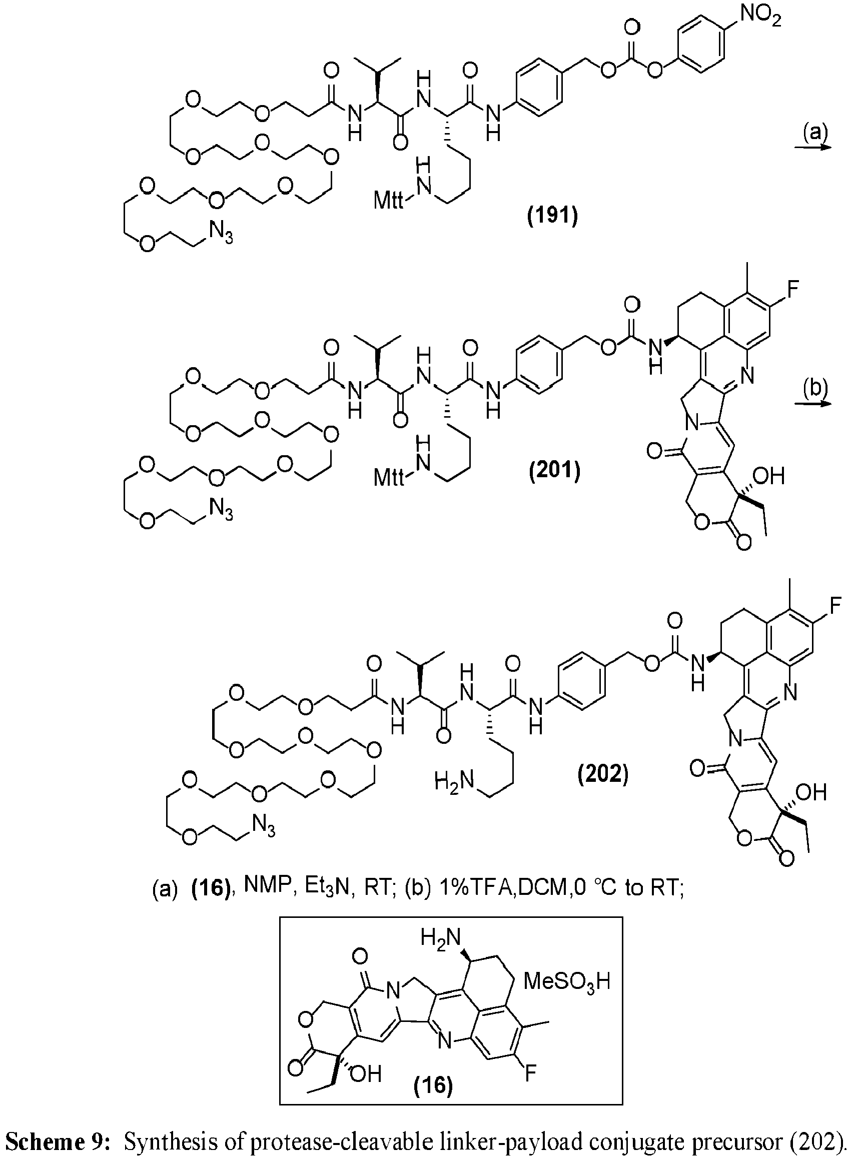

*Synthesis of 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl((1S,9S)-9-ethyl-5-fluoro-9-hydroxy-4-methyl-10,13-dioxo-2,3,9,10,13,15-hexahydro-1H,12H-benzo[de]pyram[3',4':6,7]indolizino[1,2-b]quinolin-1-yl)carbamate (201):* Triethylamine (0.09 mL, 0.62 mmol) and (1R,9R)-1-amino-9-ethyl-5-fluoro-9-hydroxy-4-methyl-1,2,3,9,12,15-hexahydro-10H,13H-benzo[de]pyrano[3',4':6,7]indolizino[1,2-b]quinoline-10,13-dione methanesulfonate (exatecan mesylate; 16; 131 mg, 0.25mmol) were added to a solution 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl (4-nitrophenyl) carbonate (191; 311 mg, 0.25 mmol) in NMP (2.5 mL) at 0 °C, and the mixture was stirred at room temperature for 8h. The progress of the reaction was monitored by LCMS. After completion of starting material, the reaction mixture was quenched with water (15 mL) and extracted with 10% methanol in chloroform (2x20 mL). The combined organic layers were dried over anhydrous sodium sulfate (Na₂SO₄) and concentrated under reduced pressure. Diethyl ether was added to the crude material, and the resulting precipitate was filtered and purified using column chromatography (Combi-Flash) eluting with 5% MeOH in DCM to provide 4-((325,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (201) as a solid (0.3 g).
LCMS: MH⁺ 1561, retention time 2.18 min.

*Synthesis of 4-((32S,35S)-35-(4-aminobutyl)-1-azido-32-isopropyl-30,33-dioxo-3,6,9,12,15,18,21,24,27-nonaoxa-31,34-diazahexatriacontan-36-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (202):* A 1% solution of trifluoroacetic acid (TFA) in DCM was added to a solution of 4-((32S,35S)-1-azido-35-(4-((diphenyl(p-tolyl)methyl)amino)butyl)-32-isopropyl-30,33-dioxo-3,6,9,12,15,18,21,24,27-nonaoxa-31,34-diazahexatriacontan-36-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (201; 300 mg, 0.19 mmol) in DCM (5 mL) at 0 °C, and the reaction mixture was stirred at room temperature for 1 h. The progress of the reaction was monitored by LCMS. After completion of starting material, the reaction mixture was concentrated under reduced pressure, and the residue was triturated with diethyl ether and purified by RP-prep-HPLC to provide 4-((32S,35S)-35-(4-aminobutyl)-1-azido-32-isopropyl-30,33-dioxo-3,6,9,12,15,18,21,24,27-nonaoxa-31,34-diazahexatriacontan-36-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (202) (70mg) as a solid. ¹H NMR (400 MHz, DMSO-*d*₆): *δ* 9.96 (s, 1H), 8.12-8.10 (q, 2H), 7.89-7.87 (d, 1H), 7.76-7.61 (d, 1H), 7.59-7.31 (m, 7H), 6.51 (s, 1H), 5.44 (s, 2H), 5.29 (s, 3H), 5.09 (s, 2H), 4.37-4.20 (m, 1H), 4.18-4.16 (t, 1H), 3.49-3.44 (m, 4H), 3.12-2.55 (m, 39H), 2.40-1.34 (m, 15H), 0.89-0.82 (m, 9H), LCMS: MH⁺ 1305, retention time 5.33 and 5.47 min.

### Synthetic Example 2: Synthesis of Targeting Ligand Precursors

Targeting ligand precursors suitable for use in the methods disclosed herein can be prepared according to the following exemplary synthetic protocols.

### Synthesis of (S)-16-(4-(((2-amino-4-oxo-3,4-dihydropteridin-6-yl)methil)amino)benzamido)-1-azido-13-oxo-3,6,9-trioxa-12-azaheptadecan-17-oic acid (606)

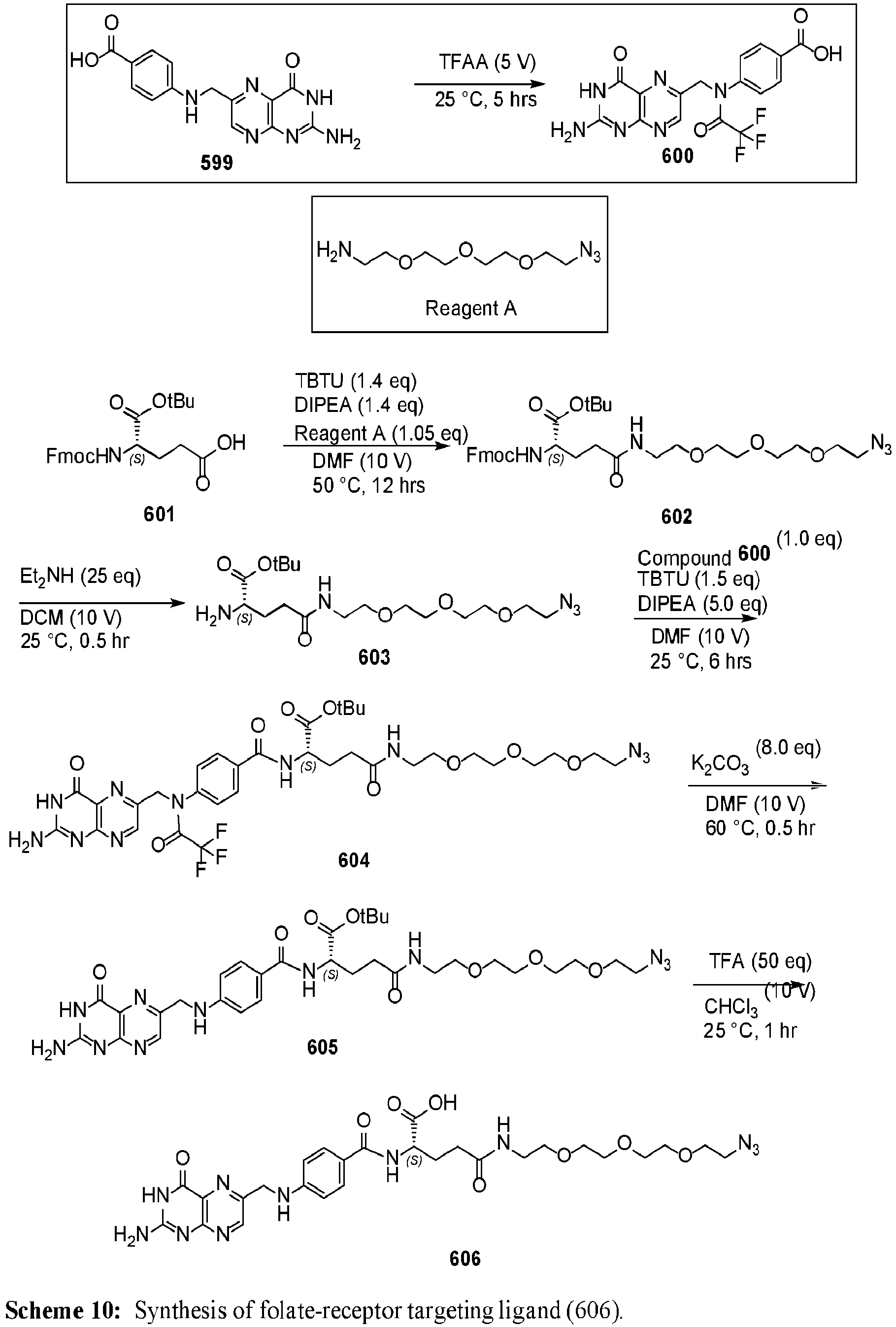

Preparation of compound 600: Compound 599 (160 g, 512 mmol) was dissolved in TFAA (800 mL) at 25 °C and stirred under a nitrogen atmosphere in the dark for 5 hrs. The solvent was then removed at 50 °C in vacuo to give the crude product. The crude product was triturated with MTBE (750 mL) for 60 min and then filtered to afford compound 600 (203 g, crude) as a solid, which was used in next step without further purification. LC-MS: ¹H NMR: (400 MHz, CDCl₃) *δ* 12.74 (br s, 1H), 8.88 (s, 1H), 7.97-8.05 (m, 2H), 7.66-7.74 (m, 2H), 5.26 (s, 1H).

Preparation of Compound 602: TBTU (238 g, 740 mmol) and DIPEA (95.7 g, 740 mmol) were added to a solution of compound 601 (225 g, 529 mmol) in DMF (2.25 L). After 30 min stirring at 20 °C, 2-(2-(2-(2-azidoethoxy)ethoxy)ethoxy)ethan-1-amine (Reagent A; 121 g, 555 mmol) was added and the mixture was stirred at 50 °C for 12 hrs. Two reaction mixtures were combined and worked up, and the residue was diluted with H₂O (3 L) and extracted with ethyl acetate (1500 mLx 3). The combined organic layers were washed with brine (800 mL x 3), dried over Na₂SO₄, filtered and concentrated under reduced pressure, and purified by column chromatography (SiO₂, Petroleum ether/Ethyl acetate = 100/1 to 1/1) to afford compound 602 (590 g) as an oil. ¹H NMR: (400 MHz, CDCl₃) *δ* 7.76-7.78 (m, 2H), 7.63-7.60 (m, 2H), 7.41-7.27 (m, 4H), 6.43 (s, 1H), 5.70 (s, 1H), 4.42-4.38 (m, 2H), 4.24-4.23 (m, 2H), 3.63-3.36 (m, 16H), 2.28-2.18 (m, 3H), 1.98-1.96 (m, 1H), 1.48 (s, 9H).

Preparation of Compound 603: N-ethylethanamine (1.27 kg, 17.4 mol) was added to a solution of compound 602 (435 g, 695 mmol) in DCM (4.35 L) and the mixture was stirred at 25 °C 3 hrs. The solvent was then removed at room temperature in vacuo, and the residue was purified by flash column chromatography (DCM/MeOH = 100/1 to 1/1) to afford compound 603 (245 g) as an oil. ¹H NMR: (400 MHz, CDCl₃) *δ* 6.55 (s, 1H), 3.67-3.30 (m, 17H), 2.34-2.30 (m, 2H), 2.10-2.06 (m, 1H), 1.87 (s, 2H), 1.77-1.73 (m, 1H), 1.44 (s, 9H). Preparation of compound 604: TBTU (119 g, 372 mmol) and DIEA (160 g, 1.24 mol) were added to a solution of compound 600 (101 g, 248 mmol) in DMF (900 mL) and the mixture was stirred for 30 minutes. Then compound 603 (100 g, 248 mmol) in DMF (100 mL) was added. The mixture was stirred at 25 °C for 12 hrs. Two reaction mixtures were combined and concentrated and the residue was diluted with H₂O (2.5 L) and extracted with ethyl acetate (1 L x 5). The combined organic layers were washed with brine (600 mL x 3), dried over Na₂SO₄, filtered and concentrated under reduced pressure to afford compound 4 (420 g, crude) as a solid, which was used in next step without further purification.

Preparation of compound 605: K₂CO₃ (585 g, 4.23 mol) was added to a solution of compound 604 (420 g, 529 mmol) in THF (4.2 mL) and H₂O (500 mL) and the mixture was stirred at 60 °C for 0.5 hr.. The reaction mixture was concentrated under reduced pressure to remove THF and the residue was diluted with H₂O (500 mL) and adjusted the pH to 3 with HCl (M=1), filtered and concentrated under reduced pressure to afford compound 605 (260 g, crude) as a solid, which was used directly without purification.

Preparation of compound 606: Trifluoroacetic acid (2.12 kg, 18.6 mol) was added in one portion to a mixture of compound 605 (260 g, 373 mmol) in CH₂Cl₂ (2.6 L) at 20 °C under nitrogen, and the mixture was stirred at 20 °C for 5 hrs. The reaction mixture was concentrated under reduced pressure and purified by HPLC (column: Agela DuraShell C18 250^{∗}80mm^{∗}10um; mobile phase: [water (10mM NH4HCO3)-MeOH]; B%: 5%-40%,20 min) to give afford compound 606 (52.5 g) as a solid. (M+H) 642.80; IR: 2107 (N₃ Bond).

The following numbered clauses are embodiments of the present disclosure:
1. A method of functionalizing a nanoparticle, comprising:
   contacting a nanoparticle with a first bifunctional precursor, wherein the first bifunctional precursor comprises a silane moiety and a diene moiety, wherein the nanoparticle comprises a surface that is reactive with a silane (e.g., a silica surface), and wherein the contacting is under conditions suitable for reaction between the silane moiety and the nanoparticle surface; and
   forming a covalent bond between the silane moiety and a surface of the nanoparticle, thereby forming a nanoparticle functionalized with a diene moiety.
2. The method of clause 1, further comprising:
   contacting the nanoparticle functionalized with a diene moiety with a second bifunctional precursor, wherein the second bifunctional precursor comprises an alkyne moiety and a group that is reactive with a diene moiety (e.g., a dienophile, e.g., maleimide), wherein the contacting is under conditions suitable for a reaction between the group that is reactive with a diene moiety and the diene moiety; and
   reacting the diene moiety of the nanoparticle with the second bifunctional precursor, thereby forming a nanoparticle functionalized with an alkyne moiety.
3. The method of clause 2, further comprising contacting the alkyne moiety with a compound comprising an alkyne-reactive group (e.g., an azide, a diene, a nitrone, or a nitrile oxide) under conditions suitable for a reaction between the alkyne moiety and the alkyne-reactive group (e.g., click chemistry conditions), thereby forming a nanoparticle functionalized with the compound (e.g., a compound comprising a payload or a targeting ligand).
4. The method of any one of the preceding clauses, wherein the diene moiety is a cyclopentadiene moiety.
5. The method of any one of the preceding clauses, wherein the first bifunctional precursor comprises the structure of Formula (A): 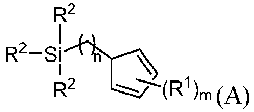 wherein
   R¹ is alkyl, alkenyl, alkynyl, heteroalkyl, cycloalkyl, heterocyclyl, aryl, heteroaryl, halo, or -OR^{A}, - wherein each alkyl, alkenyl, alkynyl, heteroalkyl, cycloalkyl, heterocyclyl, aryl, heteroaryl, may be substituted or unsubstituted;
   each R² is independently hydrogen, alkyl, halo, or -OR^{A};
   R^{A} is hydrogen, alkyl, alkenyl, alkynyl, heteroalkyl, cycloalkyl, heterocyclyl, aryl, or heteroaryl;
   n is an integer of 0 to 12; and
   m is an integer of 0 to 5.
6. The method of clause 5, wherein
   R² is -OR^{A};
   R^{A} is alkyl (e.g., ethyl);
   n is an integer of 1 to 5 (e.g., 3); and
   m is 0.
7. The method of any one of the preceding clauses, wherein the first bifunctional precursor comprises the structure of Formula (A-1): 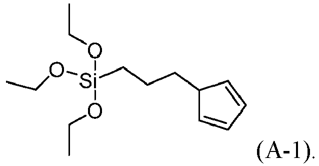
8. The method of any one of clauses 2-7, wherein the second bifunctional precursor comprises a structure of Formula (B): 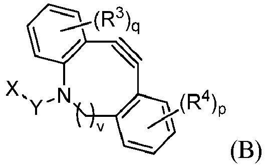 wherein:
   X is a dienophile (e.g., a moiety comprising an electron deficient alkene group; or a cyclic dienophile, e.g., a maleimide, a quinone, or maleic anhydride);
   Y is a divalent linker (e.g., a substituted or unsubstituted heteroalkylene group);
   R³ and R⁴ are each independently alkyl, alkenyl, alkynyl, heteroalkyl, cycloalkyl, heterocyclyl, aryl, heteroaryl, halo, -OR^{A}, -NR^{B}R^{C}, -NO₂, -CN, wherein each alkyl, alkenyl, alkynyl, heteroalkyl, cycloalkyl, heterocyclyl, aryl, and heteroaryl may be substituted or unsubstituted;
   R^{A}, R^{B}, and R^{C} are each independently hydrogen, alkyl, alkenyl, alkynyl, heteroalkyl, cycloalkyl, heterocyclyl, aryl, or heteroaryl;
   q and p are each independently an integer of 0 to 4; and
   v is an integer of 0 to 2.
9. The method of clause 8, wherein
   X is maleimide;
   q and p are each independently an integer of 0; and
   v is an integer of 1.
10. The method of clause 8 or 9, wherein the divalent linker is an alkylene or heteroalkylene group, wherein the alkylene or heteroalkylene group is unsubstituted or substituted (e.g., alkylene or heteroalkylene substituted with one or more oxo group).
11. The method of any one of clauses 8-10, wherein the divalent linker comprises a structure of Formula (C): 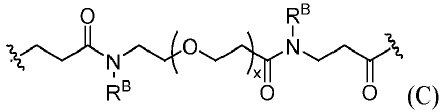
   wherein each R^{B} is independently hydrogen, alkyl, alkenyl, alkynyl, heteroalkyl, cycloalkyl, heterocyclyl, aryl, or heteroaryl;
   x is an integer of 0 to 10; and
   each N independently denotes a point of attachment to a portion of the second bifunctional precursor (e.g., an attachment to the dienophile or to the nitrogen atom of the heterocyclic ring).
12. The method of any one of clauses 8-11, wherein the second bifunctional precursor comprises a structure of Formula (B-1): 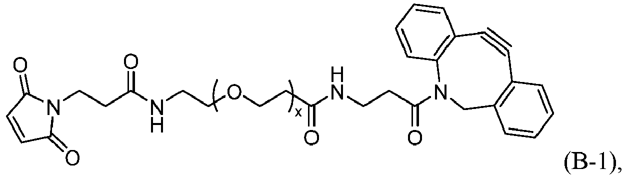 wherein x is an integer of 0 to 10 (e.g., 4).
13. The method of clause 12, wherein reacting the diene moiety with the second bifunctional precursor provides a compound of Formula (NP-1): 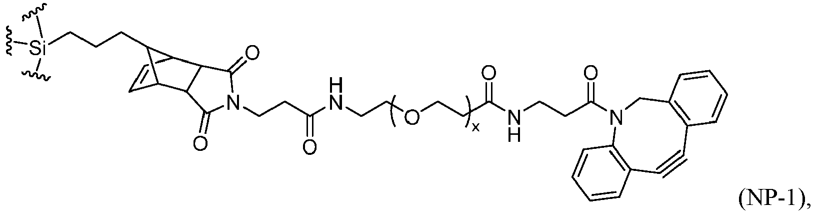 wherein x is an integer of 0 to 10 (e.g., 4), and wherein the silicon atom is a part of the nanoparticle.
14. The method of any one of clauses 3-13, wherein the compound comprising an alkyne-reactive group comprises a payload moiety (e.g., a cytotoxic drug, e.g., exatecan), or a targeting ligand (e.g., a folate receptor (FR)-targeting ligand, e.g., folic acid).
15. The method of any one of clauses 3-14, wherein the compound comprising an alkyne reactive group comprises a structure of Formula (D) or Formula (E): 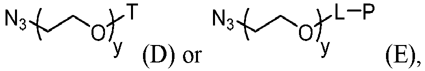 wherein:
   T is a targeting ligand (e.g., a FR-targeting ligand, e.g., folic acid);
   L is a cleavable linker moiety (e.g., a protease-cleavable linker moiety);
   P is a payload moiety (e.g., a cytotoxic drug, e.g., exatecan); and
   y is an integer of 0 to 20 (e.g., 3 or 9).
16. The method of clause 15, wherein the cleavable linker moiety comprises a structure of Formula (F): 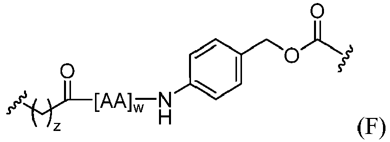 wherein
   each instance of [AA] is a natural or non-natural amino acid residue;
   z is an integer of 1 to 5;
   w is an integer of 2 or 3; and
   each 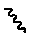 independently denotes a point of attachment to a portion of the compound comprising an alkyne-reactive group (e.g., an attachment to an azide group, or an attachment to a payload moiety).
17. The method of any one of clauses 3-16, wherein the compound comprising an alkyne reactive group comprises a structure selected from the group consisting of: 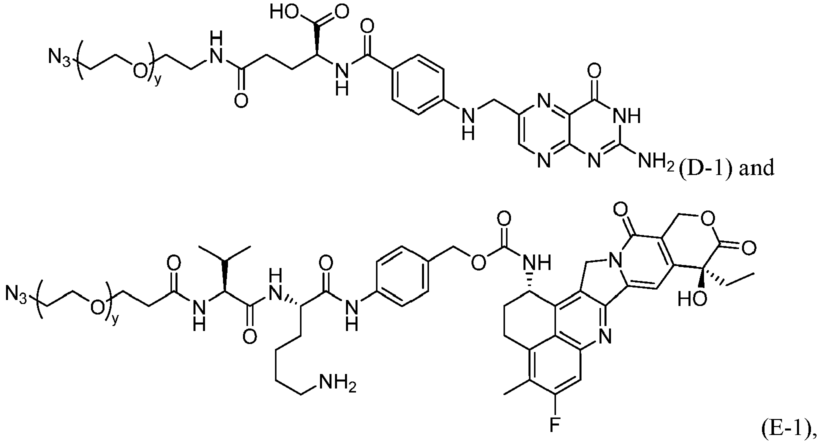 wherein y is an integer of 0 to 20.
18. The method of any one of clauses 3-17, wherein reacting the alkyne moiety with the compound comprising an alkyne-reactive group provides a compound of Formula (NP-2) or (NP-3): 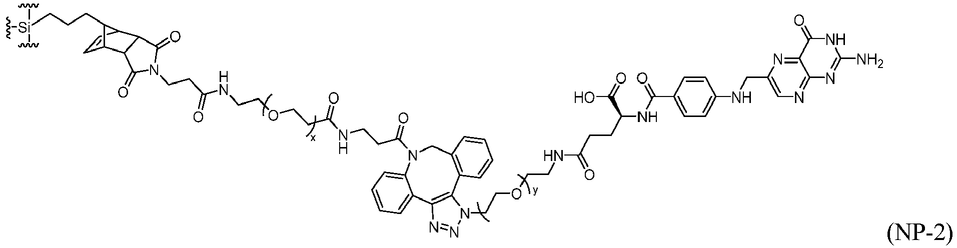 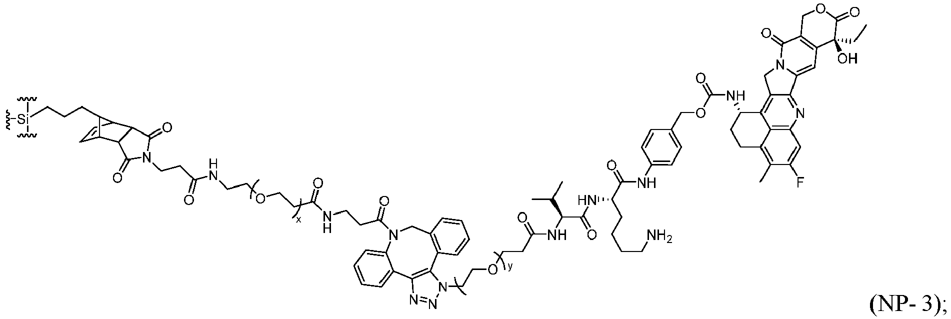 wherein:
   each instance of x is independently an integer of 0 to 10 (e.g., 4);
   each instance of y is independently an integer of 0 to 20 (e.g., 3 or 9); and
   wherein the silicon atom in each of (NP-2) and (NP-3) is a part of the nanoparticle.
19. The method of any one of the preceding clauses, wherein the nanoparticle is a silica nanoparticle (e.g., a core-shell silica nanoparticle).
20. The method of any one of the preceding clauses, wherein the nanoparticle (e.g., a surface of the nanoparticle) is coated with an organic polymer (e.g., polyethylene glycol).
21. The method of clause 20, wherein forming the covalent bond between the silane moiety of the first bifunctional precursor and the surface of the nanoparticle comprises inserting the first bifunctional precursor into the interstitial space between organic polymer molecules of the organic polymer (e.g., between PEG molecules).
22. The method of any one of the preceding clauses, wherein a nanoparticle functionalized with a plurality of diene moieties is formed.
23. The method of clause 2, wherein a nanoparticle functionalized with a plurality of alkyne moieties is formed.
24. The method of clause 23, further comprising:
   (a) reacting a first portion of the plurality of alkyne moieties with a first compound comprising an alkyne-reactive group; and
   (b) reacting a second portion of the plurality of alkyne moieties with a second compound comprising an alkyne-reactive group, thereby forming nanoparticle functionalized with the first compound and the second compound,
   wherein the first compound and the second compound are chemically distinct.
25. The method of clause 24, wherein:
   (a) the first compound is a compound of Formula (D) (e.g., a compound of Formula (D-1)) and the second compound is a compound of Formula (E) (e.g., a compound of Formula (E-1)), or
   (b) the first compound is a compound of Formula (E) and the second compound is a compound of Formula (D).
26. A method of functionalizing a silica nanoparticle, comprising:
   (i) contacting a silica nanoparticle with a first bifunctional precursor, wherein the first bifunctional precursor comprises a silane moiety and a cyclopentadiene moiety, wherein the silica nanoparticle comprises a surface that is reactive with the silane moiety (e.g., a silica surface), and wherein the contacting is under conditions suitable for reaction between the silane moiety and the silica nanoparticle surface, thereby forming a covalent bond between the silane moiety and a surface of the nanoparticle, and providing a nanoparticle functionalized with a cyclopentadiene moiety;
   (ii) contacting the nanoparticle functionalized with a cyclopentadiene moiety with a second bifunctional precursor, wherein the second bifunctional precursor comprises an alkyne moiety (e.g., DBCO) and a dienophile (e.g., maleimide), wherein the contacting is under conditions suitable for a reaction between the dienophile and the cyclopentadiene moiety, thereby reacting the diene moiety of the nanoparticle with the second bifunctional precursor, and providing a nanoparticle functionalized with an alkyne moiety; and
   (iii) contacting the alkyne moiety with a compound comprising an azide moiety, under conditions suitable for a reaction between the alkyne moiety and azide moiety (e.g., click chemistry conditions), thereby reacting the alkyne moiety of the nanoparticle with the azide moiety and providing a nanoparticle functionalized with the compound (e.g., a compound comprising a payload or a targeting ligand).
27. A method of functionalizing a silica nanoparticle, comprising:
   (i) contacting a silica nanoparticle with a first bifunctional precursor comprising a structure of Formula (A), thereby providing a nanoparticle functionalized with a cyclopentadiene moiety;
   (ii) contacting the nanoparticle functionalized with a cyclopentadiene moiety with a second bifunctional precursor comprising a structure of Formula (B), thereby providing a nanoparticle functionalized with an alkyne moiety; and
   (iii) contacting the alkyne moiety with a compound of Formula (D) or Formula (E), or both, thereby providing a nanoparticle functionalized with a targeting ligand, a payload moiety, or both.
28. The method of clause 26 or 27, wherein the first bifunctional precursor comprises the structure of Formula (A-1): 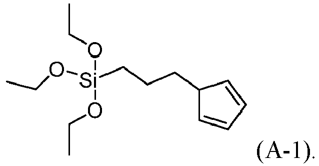
29. The method of any one of clauses 26-28, wherein the second bifunctional precursor comprises a structure of Formula (B-1): 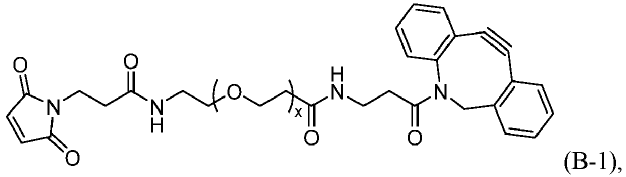 wherein x is an integer of 0 to 10 (e.g., 4).
30. The method of clause 27, wherein the compound of Formula (D) comprises a structure of Formula D-1: 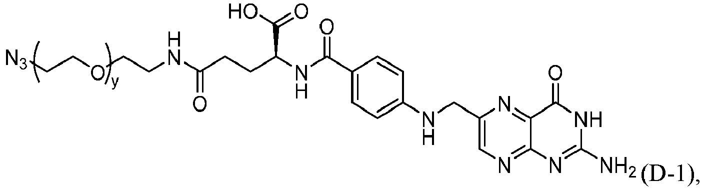 wherein y is an integer of 0 to 10 (e.g., 3).
31. The method of clause 27, wherein the compound of Formula (E) comprises a structure of Formula E-1: 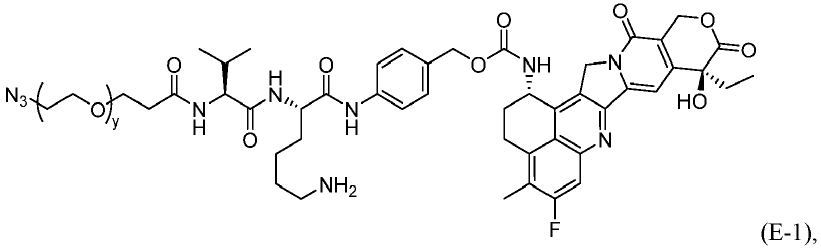 wherein y is an integer of 0 to 20 (e.g., 9).
32. The method of clause 29, wherein the method provides a nanoparticle comprising a compound of Formula (NP-2): 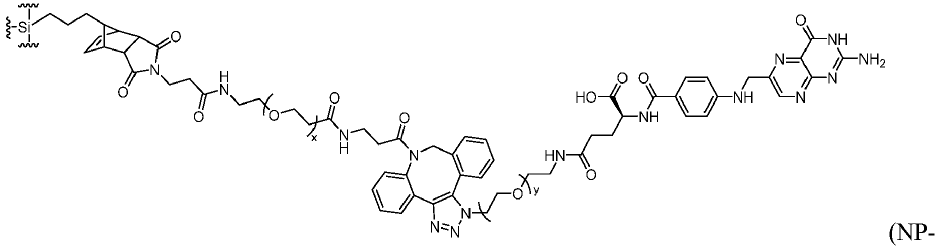 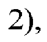 wherein x is 4 and y is 3, and the silicon atom is a part of the nanoparticle.
33. The method of clause 31, wherein the method provides a compound of Formula (NP-3): 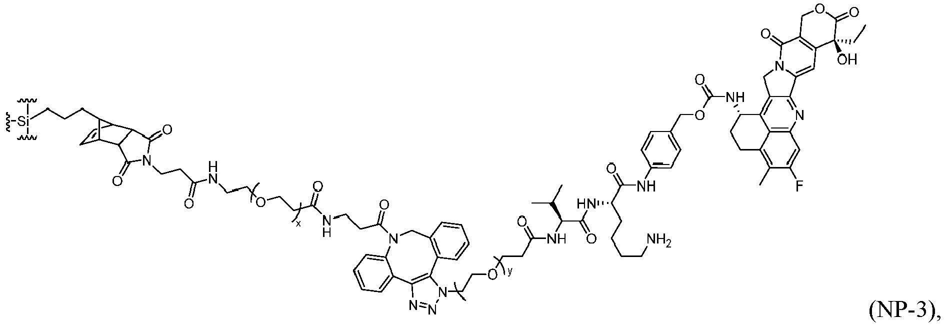 wherein x is 4 and y is 9, and the silicon atom is a part of the nanoparticle.

## Claims

1. A method of functionalizing a nanoparticle, comprising:
contacting a nanoparticle with a first bifunctional precursor comprising a silane group and another functional group (e.g., a diene, an amine, a thiol, a hydroxyl, an azide, an alkene, a nitrone, a nitrile oxide, or an alkyne), wherein the contacting is under conditions suitable for reaction between the silane group and the nanoparticle;
forming a covalent bond between the silane moiety and a surface of the nanoparticle, thereby forming a nanoparticle functionalized with a reactive group,
the method optionally further comprising contacting the nanoparticle functionalized with a reactive group with a second bifunctional precursor, wherein the second bifunctional precursor comprises a group that is reactive with the reactive group on the nanoparticle and another reactive group (e.g., a moiety that is reactive with an alkyne, azide, diene, nitrone, or nitrile oxide); and/or
optionally further comprising reacting the reactive group of the nanoparticle with the second bifunctional precursor, thereby forming a nanoparticle functionalized with the another reactive group.

2. The method of claim 1, wherein the method provides a nanoparticle comprising a plurality of the reactive groups and/or a plurality of the another reactive groups.

3. The method of claim 1 or claim 2, wherein the reactive group and the group that is reactive with the reactive group are selected from a diene and a dienophile; and/or wherein the another reactive group is an alkyne.

4. The method of any one of claims 1-3, wherein the nanoparticle (e.g., a surface of the nanoparticle) is coated with an organic polymer (e.g., polyethylene glycol); and/or wherein forming the covalent bond between the silane group of the first bifunctional precursor and a surface of the nanoparticle comprises inserting the first bifunctional precursor into the interstitial space between organic polymer molecules of the organic polymer (e.g., between PEG molecules).

5. The method of any one of claims 1-4, wherein the first bifunctional precursor comprises the structure of Formula (A): 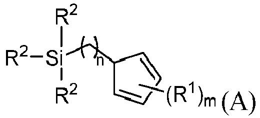 wherein
R¹ is alkyl, alkenyl, alkynyl, heteroalkyl, cycloalkyl, heterocyclyl, aryl, heteroaryl, halo, -OR^{A}, -NR^{B}R^{C}, -NO₂, or -CN, wherein each alkyl, alkenyl, alkynyl, heteroalkyl, cycloalkyl, heterocyclyl, aryl, or heteroaryl, may be substituted or unsubstituted,
each R² is independently hydrogen, alkyl, halo, or -OR^{A},
R^{A}, R^{B}, and R^{C} are each independently hydrogen, alkyl, alkenyl, alkynyl, heteroalkyl, cycloalkyl, heterocyclyl, aryl, or heteroaryl,
n is an integer of 0 to 12, and
m is an integer of 0 to 5; and/or
the second bifunctional precursor comprises a structure of Formula (B): 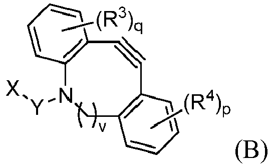 wherein:
X is a reactive group, such as a dienophile,
Y is a divalent linker,
R³ and R⁴ are each independently alkyl, alkenyl, alkynyl, heteroalkyl, cycloalkyl, heterocyclyl, aryl, heteroaryl, halo, -OR^{A}, -NR^{B}R^{C}, -NO₂, -CN, wherein each alkyl, alkenyl, alkynyl, heteroalkyl, cycloalkyl, heterocyclyl, aryl, and heteroaryl may be substituted or unsubstituted,
R^{A}, R^{B}, and R^{C} are each independently hydrogen, alkyl, alkenyl, alkynyl, heteroalkyl, cycloalkyl, heterocyclyl, aryl, or heteroaryl,
q and p are each independently an integer of 0 to 4, and
v is an integer of 0 to 2.

6. The method of any one of claims 1-5, wherein: the first bifunctional precursor comprises the structure of Formula (A-1): 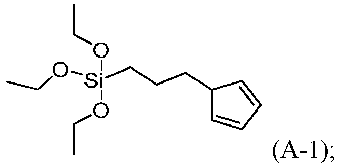 and/or
the second bifunctional precursor comprises a structure of Formula (B-1): 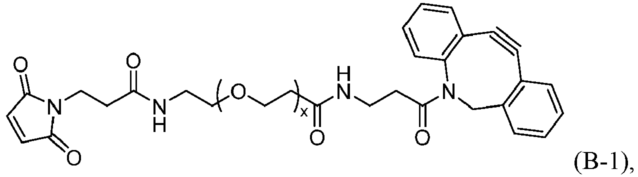
wherein x is an integer of 0 to 10 (e.g., 4).

7. The method of claim 5 or claim 6, wherein reacting the diene moiety with the second bifunctional precursor provides a compound of Formula (NP-1): 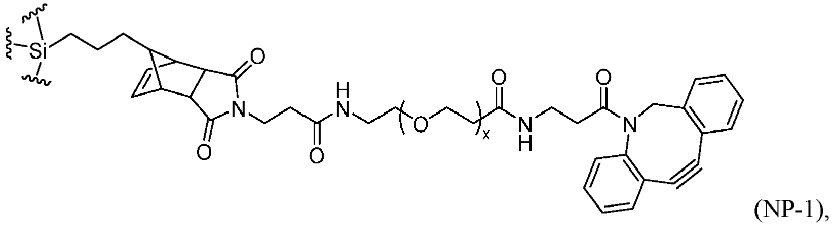 wherein x is an integer of 0 to 10 (e.g., 4), and wherein the silicon atom is a part of the nanoparticle.

8. The method of any one of claims 1-7, wherein the method provides a nanoparticle comprising one or more groups suitable for conjugation with a molecule.

9. The method of any one of claims 1-8, further comprising contacting the alkyne moiety with a compound comprising an alkyne-reactive group, thereby forming a nanoparticle functionalized with the compound.

10. The method of claim 8 or claim 9, wherein the molecule or compound comprises a payload.

11. The method of any one of claims 8-10, wherein the compound comprising an alkyne reactive group comprises a structure of Formula (D-1): 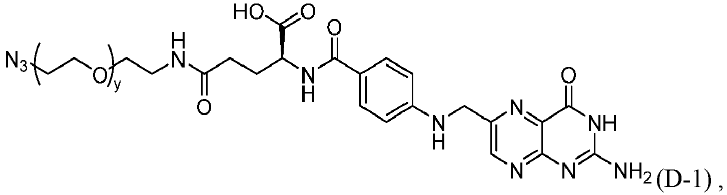 wherein y is an integer of 0 to 20.

12. The method of any one of claims 8-11, wherein reacting the alkyne moiety with the compound comprising an alkyne-reactive group provides a compound of Formula (NP-3): 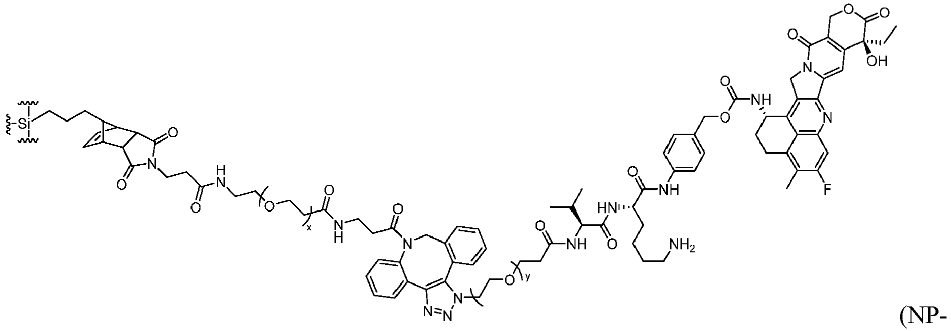 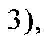 wherein x is an integer of 0 to 10 (e.g., 4), y is an integer of 0 to 20 (e.g., 3 or 9), and the silicon atom is a part of the nanoparticle.

13. The method of any one of claims 1-12, wherein the nanoparticle is an ultrasmall nanoparticle.

14. The method of any one of claims 1-13, wherein the nanoparticle has a diameter from about 0.5 nm to about 100 nm, *e.g.,* from about 0.1 nm to about 50 nm, from about 0.5 nm to about 25 nm, from about 1 nm to about 20 nm, from about 0.8 nm to about 15 nm, from about 1 nm to about 10 nm, or from about 1 nm to about 8 nm, e.g., about 1 nm, about 2 nm, about 3 nm, about 4 nm, about 5 nm, about 6 nm, about 7 nm, about 8 nm, about 9 nm, about 10 nm, about 11 nm, or about 12 nm.

15. The method of any one of claims 1-14, wherein the nanoparticle comprises a core surrounded by a shell, wherein the shell of the nanoparticle comprises silica; and/or wherein the shell is the reaction product of a silica forming compound; and/or wherein the nanoparticle is a silica nanoparticle (e.g., a core-shell silica nanoparticle).
